# EUROPEAN PATENT APPLICATION

(11) **EP 1 553 093 A1**
(43) Date of publication of application: **13.07.2005**
(21) Application number: 03761764.4
(22) Date of filing: 17.06.2003
(51) Int. Cl.: C07D 301/26, C07D 303/36, C07D 327/10

(54) **PROCESS FOR PREPARATION OF OPTICALLY ACTIVE 1-SUBSTITUTED AMINO-2,3-EPOXYPROPANES, INTERMEDIATES FOR THE SYNTHESIS THEREOF AND PROCESS FOR PREPARATION OF THE INTERMEDIATES**

(30) Priority: 28.06.2002 JP 2002189796; 22.10.2002 JP 2002306444; 22.10.2002 JP 2002306445; 13.12.2002 JP 2002362343
(71) Applicant: KANEKA CORPORATION, Osaka-shi, Osaka 530-8288 (JP)
(72) Inventor: KITAJIMA, Kumi, Aoba-ku Yokohama Kanagawa 226-0072 (JP); Nagashima, Nobuo, c/o Kaneka Corp., Takasago-shi, Hyogo 676-8688 (JP)
(74) Representative: HOFFMANN - EITLE
(86) International application number: PCT/JP2003/007695
(87) International publication number: WO 2004/002973

(57) **Abstract**

The present invention provides an industrial process for effectively and advantageously preparing optically active 1-substituted amino-2,3-epoxypropanes useful as intermediates for preparing agricultural chemicals and medical products from optically active 1-substituted amino-2,3-propanediols used as raw materials. The present invention also provides useful intermediates. Specifically, an optically active 1-substituted amino-2,3-propanediol (1) is reacted with an orthoacid ester (2) or thionyl chloride (3) to produce a cyclic optically active compound (4), and then a compound (5) containing a halogen atom is prepared by reaction with a ring-opening reaction agent having an ability to introduce a halogen atom X. Finally, an optical active 1-substituted amino-2,3-epoxypropane is prepared by ring-closure reaction in the presence of a base.

## Description

### Technical Field

The present invention relates to a process for preparation of optically active 1-substituted amino-2,3-epoxypropanes which are useful as intermediates for preparing agricultural chemicals and medical products, intermediates for the synthesis thereof, and a process for preparation of the intermediates. Optically active 1-substituted amino-2,3-epoxypropanes are very useful as intermediates for, for example, HIV protease inhibitors [Journal of the Medicinal Chemistry, Vol. 37, No. 22, 3707(1994)], and antibacterial agents having an oxazolidinone skeleton (International Publication No. 02/32857 pamphlet).

### Background Art

Known processes for preparation of optically active 1-substituted amino-2,3-epoxypropanes include the following:
(1) A process comprising protecting the amino group of aminopropanediol prepared by the reaction of optically active glycidol with ammonia, and then selectively tosylating the primary hydroxyl group of a 1,2-diol, followed by ring closure [refer to, for example, Journal of the Medicinal Chemistry, Vol. 37, No. 22, 3707 (1994) or International Publication No. 93/1174 pamphlet].
(2) A process comprising reacting azidotrimethylsilane with racemic epichlorohydrin in the presence of a catalytic amount of an optically active Cr(III) complex to produce optically active 1-azido-3-chloro-2-propanol by optical resolution, reducing and acetylating the product, and then closing the ring [refer to, for example, Tetrahedron Letters, Vol. 37, No. 44, 7939(1996)].
(3) A process comprising opening the ring of optically active 3-hydroxybutyrolactone, converting to acetonide and hydrolyzing the product to produce a butanoic acid derivative, and then performing Curtius rearrangement reaction, acetonide cleaving reaction and ring closure reaction (refer to, for example, International Publication No. 99/52855 pamphlet).
(4) A process comprising protecting the amino group of optically active 1-amino-3-chloro-2-propanol hydrochloride, and then performing ring closure reaction (refer to, for example, International Publication No. 02/32857 pamphlet).
These processes have the following disadvantages:
   In process (1), regioselectivity of selective tosylation of the primary hydroxyl group of 1,2-diol is generally not perfect, and thus tosylation of only the secondary hydroxyl group and tosylation of both hydroxyl groups occur to produce by-products, thereby disadvantageously decreasing the yield and optical purity of the resultant aminoepoxypropane derivative.
   Process (2) basically comprises optical resolution and thus produces a target product in low yield, and the formation of several types of by-products is confirmed. Thus, effective separation of the target product from the by-products is an unsolved issue. Furthermore, there is an industrial problem that a highly dangerous azide derivative is produced in the course of production.
   Process (3) has a large number of steps and uses a highly dangerous acylazide derivative in the course of production, and thus process (3) is not an industrially advantageous process.
   Process (4) is disadvantageous in that optically active 1-amino-3-chloro-2-propanol hydrochloride used as a raw material is synthesized through many steps.

As described above, all the conventional processes for producing optical active 1-substituted amino-2,3-epoxypropane have problems to be resolved as industrial production processes with high economics.

In consideration of the above-described present situation, an object of the present invention is to provide an industrially preferable process for effectively and economically producing optically active 1-substituted amino-2,3-epoxypropanes.

### Disclosure of Invention

In a first aspect of the present invention, a process for preparing an optically active 1-substituted amino-2,3-epoxypropane represented by formula (6): 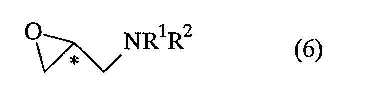 (wherein * represents an asymmetric carbon atom, R¹ and R² independently represent a hydrogen atom or a carbamate-, acyl- or aroyl-type amino protecting group, or R¹ and R² represent together an imide-type amino protecting group), comprises reacting an optically active 1-substituted amino-2,3-propanediol represented by formula (1): 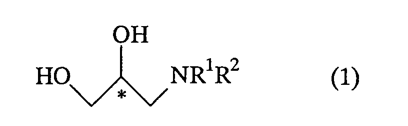 (wherein * represents an asymmetric carbon atom, and R¹ and R² represent the same as the above) with a compound represented by formula (2) or (3):

R³C(OR⁴)₃ (2)

(wherein R³ represents a hydrogen atom, an alkyl group having 1 to 6 carbon atoms, an aryl group having 6 to 10 carbon atoms, or a substituted or unsubstituted aralkyl group having 7 to 10 carbon atoms, and R⁴ represents an alkyl group having 1 to 6 carbon atoms),

SOY₂ (3)

(wherein Y represents a halogen atom or a lower alkoxy group) to produce an optically active compound represented by formula (4): 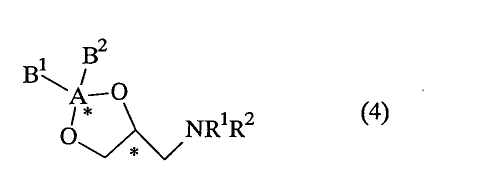 [wherein * represents an asymmetric carbon atom or an asymmetric sulfur atom, A represents a carbon atom or a sulfur atom, B¹ represents R³ (representing the same as the above), and B² represents OR⁴ (wherein R⁴ represents the same as the above) or B¹ and B² represent together an oxygen atom, and R¹ and R² represent the same as the above]; opening the ring of the compound represented by formula (4) to produce an optically active compound represented by formula (5): 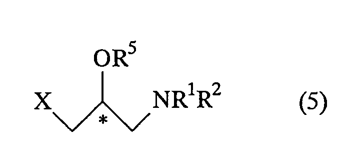 [wherein * represents an asymmetric carbon atom, X represents a halogen atom, R⁵ represents COR³ (wherein R³ represents the same as the above) or a hydrogen atom, and R¹ and R² represent the same as the above]; and further subjecting the compound represented by formula (5) to ring closure in the presence of a base.

In a second aspect of the present invention, a process for preparing an optically active compound represented by formula (4) comprises reacting an optically active 1-substituted amino-2,3-propanediaol represented by formula (1) with a compound represented by formula (2) or (3).

In a third aspect of the present invention, an optically active compound represented by formula (4) is provided.

In a fourth aspect of the present invention, a process for preparing an optically active compound represented by formula (5) comprises opening the ring of an optically active compound represented by formula (4).

In a fifth aspect of the present invention, a process for preparing an optically active compound represented by formula (5) comprises reacting an optically active 1-substituted amino-2,3-propanediol represented by formula (1) with a compound represented by formula (2) or (3) to produce an optically active compound represented by formula (4), and then opening the ring of the compound.

The present invention will be described in further detail below.

The present invention comprises the three steps of converting an optically active 1-substituted amino-2,3-propanediol represented by formula (1) to an optically active compound represented by formula (4), converting optically active compound (4) to an optically active compound represented by formula (5) by ring opening, and converting the optically active compound (5) to an optically active 1-substituted amino-2,3-epoxypropane represented by formula (6) by ring closure. Each of the steps will be described below.

In the first step, the optically active 1-substituted amino-2,3-propanediol represented by formula (1) used as a starting material is converted to the optically active compound (4). First, compound (1) used as the starting material is descried.

Compound (1), i.e., the optically active 1-substituted amino-2,3-propanediol, can be produced according to a known method, for example, a method of converting an optically active 1-chloro-2,3-propanediol to an optically active 1-amino-2,3-propanediol by reaction with ammonia water in the presence of ammonium chloride (Japanese Unexamined Patent Application Publication No. 3-41056), and then protecting the amino group, or a method of converting an optically active glycerol to its acetonide-protected compound and then being derivatized to phthalimide-protected 1-amino-2,3-propanediol by Mitsunobu reaction (Tetrahedron Asymmetry), Vol. 7, No. 8, p. 2411 (1996)).

The reaction for protecting the amino group can be performed by, for example, the method described in Protective Groups In Organic Synthesis Third Edition, Green, T. W., Wuts, P. G. M, Jhon Wiley & Sons, pp. 494-572.

The optically active 1-chloro-2,3-propanediol may have either a (R) or (S) configuration, and thus the resultant optically active 1-substituted amino-2,3-propanediol represented by formula (1) also has a (R) or (S) configuration. The present invention includes optically active 1-substituted amino-2,3-propanediol having the (R) configuration and optically active 1-substituted amino-2,3-propanediol having the (S) configuration represented by formula (1).

In the optically active 1-substituted amino-2,3-propanediol represented by formula (1), R¹ and R² independently represent a hydrogen atom or a carbamate-, acyl-type, or aroyl-type amino-protecting group, or R¹ and R² represent together an imide-type amino-protecting group.

Examples of the carbamate-type amino-protecting group include methoxycarbonyl, ethoxycarbonyl, n-propoxycarbonyl, isopropoxycarbonyl, tert-butoxycarbonyl, pentyloxycarbonyl, isopentyloxycarbonyl, cyclohexyloxycarbonyl, 2-chloroethoxycarbonyl, 2-iodoethoxycarbonyl, 2,2,2-trichloroethoxycarbonyl, benzhydryloxycarbonyl, bis-(4-methoxyphenyl)methoxycarbonyl, phenacyloxycarbonyl, 2-trimethylsilylethoxycarbonyl, 2-triphenylsilylethoxycarbonyl, vinyloxycarbonyl, 2-propenyloxycarbonyl, 2-chloro-2-propenyloxycarbonyl, 3-methoxycarbonyl-2-propenyloxycarbonyl, 2-methyl-2-propenyloxycarbonyl, 2-butenyloxycarbonyl, cinnamyloxycarbonyl, phenoxycarbonyl, benzyloxycarbonyl, 4-bromobenzyloxycarbonyl, 2-chlorobenzyloxycarbonyl, 3-chlorobenzyloxycarbonyl, 3,5-dimethoxybenzyloxycarbonyl, 4-methoxybenzyloxycarbonyl, 2-nitrobenzyloxycarbonyl, 4-nitrobenzyloxycarbonyl, 2-nitro-4,5-dimethoxybenzyloxycarbonyl, 3,4,5-trimethoxybenzyloxycarbonyl, and phenethyloxycarbonyl.

Examples of the acyl-type amino-protecting group include formyl, acetyl, propionyl, butyryl, pivaloyl, 2-chloroacetyl, 2-bromoacetyl, 2-iodoacetyl, and 2,2-dichloroacetyl.

Examples of the aroyl-type amino-protecting group include benzoyl, 4-methoxybenzoyl, 3-hydroxy-2-methylbenzoyl, 3-acetoxy-2-methylbenzoyl, 4-nitrobenzoyl, and naphthylcarbonyl.

Examples of the imide-type amino-protecting group include phthaloyl, tetrachlorophthaloyl, and 4-nitrophthaloyl.

From the viewpoint of industrial availability and economics of a protecting group introducing agent, the protecting group is preferably methoxycarbonyl, ethoxycarbonyl, tert-butoxycarbonyl, benzyloxycarbonyl, acetyl, benzoyl, or phthaloyl, and more preferably tert-butoxycarbonyl, benzyloxycarbonyl, benzoyl, or phthaloyl.

Next, the conversion of compound (1) to optically active compound (4) will be described.

Compound (1) is converted to optically active compound (4) which is an intermediate compound suitable for preparing the optically active 1-substituted amino-2,3-epoxypropane represented by formula (6), which is a target compound of the present invention.

The conversion reaction is performed by, for example, reaction of compound (1) with a compound represented by formula (2) or (3).

The conversion using each of compound (2) and compound (3) will be described.

When compound (2) is used, in formula (2), R³ represents a hydrogen atom, an alkyl group having 1 to 6 carbon atoms, an aryl group having 6 to 10 carbon atoms, or a substituted or unsubstituted aralkyl group having 7 to 10 carbon atoms, and these groups may be linear, branched, or cyclic.

Such groups are not particularly limited. Examples of an alkyl group having 1 to 6 carbon atoms include methyl, ethyl, n-propyl, isopropyl, n-butyl, isobutyl, tert-butyl, pentyl, cyclopentyl, hexyl, and cyclohexyl. Examples of an aryl group having 6 to 10 carbon atoms include phenyl, 4-methylphenyl, 2,4,6-trimethylphenyl, 1-naphthyl, and 2-naphthyl. Examples of a substituted or unsubstituted aralkyl group having 7 to 10 carbon atoms include benzyl, 1-phenethyl, 2-phenethyl, and 1-(4-methoxyphenyl)ethyl.

From the viewpoint of availability of compound (2), R³ is preferably hydrogen atom, methyl, ethyl, n-propyl, or phenyl.

In the compound represented by formula (2), R⁴ represents an alkyl group having 1 to 6 carbon atoms, which may be linear, branched, or cyclic.

Although such a group is not particularly limited, examples of the group include methyl, ethyl, n-propyl, isopropyl, n-butyl, isobutyl, tert-butyl, pentyl, cyclopentyl, hexyl, and cyclohexyl. From the viewpoint of availability of compound (2), R⁴ is preferably methyl or ethyl.

Therefore, the compound represented by formula (2) is preferably an orthoformate, an orthoacetate, an orthopropionate, an orthobutylate, or an orthobenzoate. Specifically, the compound is trimethyl orthoformate, triethyl orthoformate, trimethyl orthoacetate, triethyl orthoacetate, trimethyl orthopropionate, triethyl orthopropionate, trimethyl orthobutylate, triethyl orthobutylate, trimethyl orthobenzoate, or triethyl orthobenzoate.

In the optically active compound represented by formula (4) and produced by reaction of the optically active 1-substituted amino-2,3-propanediol represented by formula (1) with the compound represented by formula (2), A represents a carbon atom, B¹ represents R³, B² represents OR⁴, and R³ and R⁴ are defined as described above. R¹ and R² are also defined as described above. However, in order to produce the optically active compound (5) described below in high yield, more preferably, one of R¹ and R² is a hydrogen atom, and the other is a carbamate-type amino-protecting group. The compound represented by formula (4) in which one of R¹ and R² is a hydrogen atom, and the other is a carbamate-type amino-protecting group is a novel compound. In producing the optically active compound (4) by reaction of compound (1) with compound (2), a new asymmetric carbon atom is formed. However, the stereoselectivity at the newly formed asymmetric carbon atom is generally low, and thus both asymmetric carbon atoms each having the (R) or (S) configuration are formed without a deviation to one of the configurations. Namely, when an optically pure (R)- or (S)-1-substituted amino-2,3-propanediol represented by formula (1) is used, a mixture of substantially equal amounts of two diastereomers is produced. When an optically impure (R)- or (S)-1-substituted amino-2,3-propanediol represented by formula (1) is used, a mixture of four diastereomers is produced due to the reflection of the optical purity of the optically active 1-substituted amino-2,3-propanediol represented by formula (1). The present invention includes both cases.

The reaction for producing the optically active compound represented by formula (4) is performed in the presence of an acid catalyst. Although the acid catalyst used is not particularly limited, a proton acid, a salt of a proton acid with an amine, or a Lewis acid can be preferably used. Examples of the proton acid include hydrogen halides such as hydrogen chloride, hydrogen bromide, and hydrogen iodide; inorganic acids such as sulfuric acid, phosphoric acid, perchloric acid, and hydrofluoroboric acid; organic or inorganic sulfonic acids such as methanesulfonic acid, benzenesulfonic acid, p-toluenesulfonic acid, trifluoromethanesulfonic acid, camphorsulfonic acid, and chlorosulfonic acid; and organic carboxylic acids such as formic acid, acetic acid, butyric acid, chloroacetic acid, dichloroacetic acid, trichloroacetic acid, bromoacetic acid, dibromoacetic acid, trifluoroacetic acid, glycolic acid, oxalic acid, succinic acid, benzoic acid, and salicylic acid. The salt of the proton acid with the amine is preferably a salt of a strong proton acid such as a hydrogen halide or sulfonic acid with an amine. Examples of the amine include aliphatic primary, secondary and tertiary amines, ammonia, and heterocyclic amines.

Examples of such a salt include ammonium chloride, ammonium bromide, ammonium sulfate, ammonium nitrate, pyridine p-toluenesulfonate, quinoline p-toluenesulfonate, triethylamine sulfate, triethylamine hydrochloride, pyridine hydrochloride, imidazole hydrochloride, methylamine sulfate, dimethylamine sulfate, pyridine sulfate, lutidine sulfate, collidine sulfate, pyridine trifluoromethanesulfonate, pyridine trifluoroacetate, and pyridine trichloroacetate. The salt may be directly added, or the proton acid and the amine may be mixed to prepare the salt in the reaction system. Examples of the Lewis acid include zinc chloride, zinc bromide, zinc iodide, aluminum chloride, aluminum bromide, a boron trifluoride-ether complex, stannic chloride, ferric chloride, and titanium tetrachloride. Alternatively, a solid acid such as a cationic exchange resin, e.g., Dowex 50, silica gel, polyphosphoric acid, or phosphorus pentoxide can be used as the catalyst. Among these acids, particularly, acetic acid, p-toluenesulfonic acid, pyridine p-toluenesulfonate, triethylamine sulfate, zinc chloride or zinc bromide is preferably used.

The amount of the acid used is not particularly limited. The lower and upper limits of the amount are generally 0.01 mol% and 100 mol%, respectively, relative to the optically active 1-substituted amino-2,3-propanediol represented by formula (1). In view of the reaction rate and economics, the lower and upper limits are preferably 0.1 mol% and 10 mol%, respectively.

Compound (2) is usually used in excess relative to the optically active 1-substituted amino-2,3-propanediol represented by formula (1). The lower and upper limits of the amount of compound (2) are generally 100 mol% and 500 mol%, respectively. In view of the reaction rate and economics, the lower and upper limits are preferably 105 mol% and 300 mol%, respectively.

In the above-described reaction, a solvent is generally used, and the solvent is not particularly limited unless the reaction is inhibited. Examples of the solvent include aliphatic or alicyclic hydrocarbon solvents such as heptane, hexane, cyclohexane, and methylcyclohexane; aromatic hydrocarbon solvents such as benzene, toluene, and xylene; ether solvents such as diethyl ether, diisopropyl ether, and methyl tert-butyl ether; ester solvents such as methyl acetate, ethyl acetate, and butyl acetate; ketone solvents such as acetone and ethyl methyl ketone; halogenated hydrocarbon solvents such as methylene chloride, 1,2-dichloroethane, chloroform, carbon tetrachloride, and chlorobenzene; and nitrile solvents such as acetonitrile, propionitrile, and benzonitrile. These solvents may be used in combination of two or more. Preferably, toluene or methylene chloride is used.

The amount of the solvent used is not particularly limited. The lower and upper limits of the amount are 1 ml/g and 100 ml/g, respectively, relative to the optically active 1-substituted amino-2,3-propanediol represented by formula (1). In view of the reaction operationality and economics, the lower and upper limits are preferably 2 ml/g and 30 ml/g, respectively.

The reaction temperature depends on the types of the solvent and acid catalyst, but any temperature can be selected in the range from the freezing point to the boiling point of the reaction solvent used. The lower and upper limits of the reaction temperature are generally -20°C and 150° C, and preferably 0° C and 115° C, respectively.

Although the reaction time depends on the types of compound (2) and the acid catalyst, the reaction solvent, and the reaction temperature, the time is generally about 0.5 to 82 hours.

The degree of the reaction progress can be confirmed by an ordinary analytical means, for example, time-lapse analysis of the reaction solution using high-performance liquid chromatography (HPLC). The completion of the reaction can be confirmed by the disappearance of the optically active 1-substituted amino-2,3-propanediol represented by formula (1).

A post-treatment after the reaction is not particularly limited. However, after the completion of the reaction, for example, ammonia water or water may be added to the reaction solution to terminate the reaction, and the target product may be extracted with an appropriate organic solvent such as ethyl acetate, toluene, benzene, diethyl ether, or dichloromethane. Then, the extract may be washed with water and saturated brine or the like and subjected to concentration of the solvent to isolate the target product. Alternatively, the reaction solvent may be distilled off from the reaction solution under reduced pressure to isolate the target product as a concentrate, or the reaction solvent may be partially distilled off to produce a concentrated reaction solution containing the target product which may be used in a next step. If required, the target product may be isolated and purified by, for example, column chromatography.

When the compound represented by formula (3) is used, Y in formula (3) represents a halogen atom or a lower alkoxy group. Examples of a halogen atom include a fluorine atom, a chlorine atom, a bromine atom, and an iodine atom.
Examples of a lower alkoxy group include methoxy, ethoxy, n-propoxy, isopropoxy, n-butoxy, sec-butoxy, and tert-butoxy. In view of the availability of compound (3), the halogen atom is preferably a chlorine or bromine atom, and the lower alkoxy group is preferably methoxy, ethoxy, n-propoxy, or isopropoxy.

Therefore, examples of the compound represented by formula (3) include thionyl fluoride, thionyl chloride, thionyl bromide, thionyl iodide, dimethyl sulfite, diethyl sulfite, di-n-propyl sulfite, diisopropyl sulfite, di-n-butyl sulfite, di-sec-butyl sulfite, and di-tert-butyl sulfite. In particular, thionyl chloride, thionyl bromide, dimethyl sulfite, diethyl sulfite, di-n-propyl sulfite and diisopropyl sulfite are preferred.

In the optically active compound represented by formula (4) and produced by reaction of the optically active 1-substituted amino-2,3-propanediol represented by formula (1) with the compound represented by formula (3), A represents a sulfur atom, B¹ and B² represent together an oxygen atom. R¹ and R² are defined as described above. The optically active compound represented by formula (4) in which R¹ and R² independently represent a hydrogen atom or a carbamate-type, acyl-type or aroyl-type amino-protecting group is a novel compound.

The novel optically active compound (4) is a very important intermediate for producing, for example, an intermediate of a HIV protease inhibitor [Journal of the Medicinal Chemistry, Vol. 37, No. 22, 3707 (1994)], or an intermediate of an antibacterial agent having an oxazolidinone skeleton (International Publication No. 02/32857 pamphlet). By producing the novel optically active compound (4), the optically active 1-substituted amino-2,3-epoxypropane represented by formula (6) can be effectively and advantageously produced by an industrial process.

In producing the optically active compound (4) by reaction of compound (1) with compound (3), a new asymmetric sulfur atom is formed. However, the stereoselectivity at the newly formed asymmetric sulfur atom is generally low, and thus both asymmetric sulfur atoms each having the (R) or (S) configuration are formed without a deviation to one of the configurations. Namely, when an optically pure (R)- or (S)-1-substituted amino-2,3-propanediol represented by formula (1) is used, a mixture of substantially equal amounts of two diastereomers is produced. When an optically impure (R)- or (S)-1-substituted amino-2,3-propanediol represented by formula (1) is used, a mixture of four diastereomers is produced due to the reflection of the optical purity of the optically active 1-substituted amino-2,3-propanediol represented by formula (1). The present invention includes both cases.

Compound (3) is usually used in excess relative to the optically active 1-substituted amino-2,3-propanediol represented by formula (1). The lower and upper limits of the amount of compound (3) are 100 mol% and 1,000 mol%, respectively. In view of the reaction rate and economics, the lower and upper limits are preferably 105 mol% and 300 mol%, respectively.

In the above-described reaction, a solvent is generally used, and the solvent is not particularly limited unless the reaction is inhibited. Examples of the solvent include aliphatic or alicyclic hydrocarbon solvents such as heptane, hexane, cyclohexane, and methylcyclohexane; aromatic hydrocarbon solvents such as benzene, toluene, and xylene; ether solvents such as diethyl ether, diisopropyl ether, methyl tert-butyl ether, tetrahydrofuran, and dioxane; ester solvents such as methyl acetate, ethyl acetate, and butyl acetate; ketone solvents such as acetone and ethyl methyl ketone; halogenated hydrocarbon solvents such as methylene chloride, 1,2-dichloroethane, chloroform, carbon tetrachloride, and chlorobenzene; nitrile solvents such as acetonitrile, propionitrile, and benzonitrile; N,N-dimethylformamide; and N,N-dimethylacetamide. These solvents may be used in combination of two or more. Preferably, toluene or methylene chloride is used. The reaction may also be performed without using the solvent.

The amount of the solvent used is not particularly limited. The lower and upper limits of the amount are 1 ml/g and 100 ml/g, respectively, relative to the optically active 1-substituted amino-2,3-propanediol represented by formula (1). In view of the reaction operationality and economics, the lower and upper limits are preferably 2 ml/g and 30 ml/g, respectively.

When Y is a halogen atom, for example, an amine may be added for neutralizing the hydrogen halide produced in the reaction. However, the reaction can be performed without the addition of an amine or the like. In particular, when the optically active 1-substitued amino-2,3-propanediol represented by formula (1) has a functional group weak against the produced hydrogen halide, an amine is preferably added. Although the amine is not particularly limited, the amine is preferably a tertiary amine such as trimethylamine, triethylamine, or tripropylamine, or pyridine.

The lower and upper limits of the amount of the amine added are 200 mol% and 2000 mol%, respectively, relative to the compound represented by formula (1). In view of economics, the lower and upper limits are preferably 200 mol% and 600 mol%, respectively.

When Y is a lower alkoxy group, an acid catalyst or base catalyst may be added as occasion demands. Examples of the acid catalyst include organic sulfonic acids such as methanesulfonic acid, benzenesulfonic acid, p-toluenesulfonic acid, trifluoromethanesulfonic acid, and camphorsulfonic acid. Examples of the base catalyst include lithium tert-butoxide and sodium tert-butoxide.

The lower and upper limits of the amount of the acid catalyst added are 1 mol% and 50 mol%, and preferably 1 mol% and 25 mol%, respectively, relative to the optically active 1-substituted amino-2,3-propanediol represented by formula (1). In the use of the base catalyst, the lower and upper limits of the amount of the catalyst added are 1 mol% and 50 mol%, and preferably 1 mol% and 10 mol%, respectively.

When Y is a lower alkoxy group, the alcohol produced from the alkoxy group with progress of the reaction is preferably distilled off to shift the reaction equilibrium to the product side according to demand.

The reaction temperature depends on the types of the solvent and acid catalyst used, but any temperature can be selected in the range from the freezing point to the boiling point of the reaction solvent used. The lower and upper limits of the reaction temperature are generally 0°C and 150°C, and preferably 0° C and 50°C, respectively.

Although the reaction time depends on the types of the optically active 1-substituted amino-2,3-propanediol represented by formula (1) and compound (3), the reaction solvent, and the reaction temperature, the time is generally about 0.5 to 24 hours.

The degree of reaction progress can be determined by an ordinary analytical means, for example, time-lapse analysis of the reaction solution using high-performance liquid chromatography (HPLC). The completion of the reaction can be confirmed by the disappearance of the optically active 1-substituted amino-2,3-propanediol represented by formula (1).

A post-treatment after the reaction is not particularly limited. However, after the completion of the reaction, for example, water may be added to the reaction solution to terminate the reaction, and the target product may be extracted with an appropriate organic solvent such as ethyl acetate, toluene, benzene, diethyl ether, or dichloromethane. Then, the extract may be washed with water and diluted hydrochloric acid or saturated brine and subjected to concentration of the solvent to isolate the target product. Alternatively, the reaction solvent may be distilled off from the reaction solution under reduced pressure to isolate the target product as a concentrate. If required, the target product may be isolated and purified by, for example, column chromatography.

Next, the second step will be described.

In this step, the optically active compound represented by formula (4) and prepared as described above is converted to the optically active compound represented by formula (5) by ring opening. The ring-opening reaction for preparing compound (4) by reaction of the optically active 1-substituted amino-2,3-propanediol represented by formula (1) with the compound represented by formula (2) is different from that for preparing compound (4) by reaction with the compound represented by formula (3). Therefore, both cases will be described separately.

When compound (4) is prepared by reaction of compound (1) with compound (2), in optically active compound (5) produced by ring opening of compound (4), X represents a halogen atom, for example, fluorine atom, chlorine atom, bromine atom, or iodine atom, and preferably chlorine atom or bromine atom, R⁵ represents COR³, and R³, R¹, and R² are defined as described above. In order to prepare the optically active compound (5) in high yield, preferably, one of R¹ and R² is a hydrogen atom, and the other is a carbamate-type amino-protecting group. The optically active compound (5) in which one of R¹ and R² is a hydrogen atom, the other is a carbamate-type amino-protecting group, and R³ is a hydrogen atom or an aryl group having 6 to 10 carbon atoms is a novel compound.

The optically active compound represented by formula (5) has an asymmetric carbon atom having a configuration which is derived from that of the corresponding asymmetric carbon atom of the optically active compound represented by formula (4). In the step of ring-opening reaction, the asymmetric carbon atom is not involved in the reaction, and thus the configuration of the corresponding asymmetric carbon atom of the optically active compound represented by formula (4) is not changed and is inherited to the configuration of the asymmetric carbon atom of the optically active compound represented by formula (5).

As an agent used in the step of ring-opening reaction, an agent having the ability to introduce a halogen atom X into the optically active compound represented by formula (4) by substitution reaction is used. Examples of such an agent include trimethylsilyl chloride, trimethylsilyl bromide, trimethylsilyl iodide, acetyl chloride, acetyl bromide, acetyl iodide, thionyl chloride, thionyl bromide, sulfuryl chloride, phosphorus trichloride, phosphorus tribromide, phosphorus pentachloride, phosphorus oxychloride, trityl chloride, hydrogen chloride, a hydrogen bromide-acetic acid solution, and diethylaminosulfur trifluoride (DAST). Preferably, the agent is trimethylsilyl chloride, acetyl chloride, thionyl chloride, sulfuryl chloride, phosphorus pentachloride, phosphorus oxychloride, or a hydrogen bromide-acetic acid solution.

When the optically active compound represented by formula (4) and/or the optically active compound represented by formula (5) is sensitive to an acid, for example, a base such as triethylamine may be added in an amount up to 10 mol% so that the reaction can be efficiently performed while inhibiting decomposition of the optically active compound represented by formula (4) and/or the optically active compound represented by formula (5) in the ring-opening reaction. When the reaction rate is low, for example, a Lewis acid such as zinc chloride, zinc bromide, or tin bromide, or sodium iodide may be added to improve the reaction rate.

The lower and upper limits of the amount of the ring-opening reaction agent used are 100 mol% and 500 mol%, and preferably 110 mol% and 150 mol%, respectively, relative to the optically active compound represented by formula (4).

In the above-described reaction, a solvent is generally used, and the solvent is not particularly limited unless the reaction is inhibited. Examples of the solvent include aliphatic or alicyclic hydrocarbon solvents such as heptane, hexane, cyclohexane, and methylcyclohexane; aromatic hydrocarbon solvents such as benzene, toluene, and xylene; ether solvents such as diethyl ether, diisopropyl ether, and methyl tert-butyl ether; ester solvents such as methyl acetate, ethyl acetate, and butyl acetate; ketone solvents such as acetone and ethyl methyl ketone; halogenated hydrocarbon solvents such as methylene chloride, 1,2-dichloroethane, chloroform, carbon tetrachloride, and chlorobenzene; nitrile solvents such as acetonitrile, propionitrile, and benzonitrile. These solvents may be used in combination of two or more. As the reaction solvent in the step, the solvent used in preparing the optically active compound represented by formula (4) can be directly used, or another solvent can be selected. However, in the viewpoint of efficiency and economics of the process, of course, the solvent used in preparing the optically active compound represented by formula (4) is preferably directly used. Among these solvents, toluene and methylene chloride are preferred.

Although the amount of the solvent used is not particularly limited, the lower and upper limits of the amount are 1 ml/g and 100 ml/g, respectively, relative to the optically active compound represented by formula (4). In view of the reaction operationality and economics, the lower and upper limits are preferably 2 ml/g and 20 ml/g, respectively.

The reaction temperature depends on the types of the solvent used and optically active compound used and represented by formula (4), but any temperature can be selected in the range from the freezing point to the boiling point of the reaction solvent used. The lower and upper limits of the reaction temperature are generally -20°C and 150° C, and preferably 0° C and 115° C, respectively.

Although the reaction time depends on the type of the optically active compound used and represented by formula (4), the reaction solvent, and the reaction temperature, the time is generally about 0.5 to 72 hours.

The degree of reaction progress can be determined by an ordinary analytical means, for example, time-lapse analysis of the reaction solution by high-performance liquid chromatography (HPLC). The completion of the reaction can be confirmed by the disappearance of the optically active compound represented by formula (4).

A post-treatment after the reaction is not particularly limited. However, after the completion of the reaction, for example, saturated aqueous sodium bicarbonate may be added to the reaction solution under cooling to terminate the reaction. Then, the target product may be extracted with an appropriate organic solvent such as ethyl acetate, toluene, benzene, diethyl ether, or dichloromethane. The extract may be washed with water and saturated brine and then concentrated to isolate the target product. Alternatively, the reaction solvent may be distilled off from the reaction solution under reduced pressure to isolate the target product as a concentrate, or the reaction solvent may be partially distilled off to prepare a concentrate solution of the reaction solvent containing the target product. The concentrate solution may be used in a next step. If required, the target product may be isolated and purified by, for example, column chromatography.

When compound (4) is prepared by the reaction of compound (1) with compound (3), in optically active compound (5) produced by ring opening of compound (4), X represents a halogen atom, as described above, R⁵ represents a hydrogen atom, and R¹ and R² are defined as above.

The optically active compound represented by formula (5) has an asymmetric carbon atom having a configuration which is derived from that of the corresponding asymmetric carbon atom of the optically active compound represented by formula (4). Similar to the aforementioned step, in this step of ring-opening reaction, the asymmetric carbon atom is not involved in the reaction, and thus the configuration of the corresponding asymmetric carbon atom of the optically active compound represented by formula (4) is not changed and is inherited to the configuration of the asymmetric carbon atom of the optically active compound represented by formula (5).

The agent used in the step of ring-opening reaction is an agent having the ability to introduce a halogen atom X into the optically active compound represented by formula (4) by substitution reaction. Examples of such an agent include lithium fluoride, lithium chloride, lithium bromide, lithium iodide, sodium bromide, and sodium iodide. Among these compounds, lithium chloride, lithium bromide, and sodium bromide are preferred.

The lower and upper limits of the amount of the ring-opening reaction agent used are 100 mol% and 1500 mol%, and preferably 150 mol% and 600 mol%, respectively, relative to the optically active compound represented by formula (4).

In the above-described reaction, a solvent is generally used, and the solvent is not particularly limited unless the reaction is inhibited. Examples of the solvent include ether solvents such as diethyl ether, diisopropyl ether, methyl tert-butyl ether, tetrahydrofuran, dioxane, and 1,2-dimethoxyethane; ester solvents such as methyl acetate, ethyl acetate, and butyl acetate; ketone solvents such as acetone and ethyl methyl ketone; halogenated hydrocarbon solvents such as methylene chloride, 1,2-dichloroethane, chloroform, carbon tetrachloride, and chlorobenzene; nitrile solvents such as acetonitrile, propionitrile, and benzonitrile; N,N-dimethylformamide; N,N-dimethylacetamide; and dimethylsulfoxide. These solvents may be used in combination of two or more. Among these solvents, aprotic polar solvents such as acetone, tetrahydrofuran, 1,2-dimethoxyethane, acetonitrile, and N,N-dimethylformamide are preferred.

Although the amount of the solvent used is not particularly limited, the lower and upper limits of the amount are 1 ml/g and 100 ml/g, respectively, relative to the optically active compound represented by formula (4). In view of the reaction operationality and economics, the lower and upper limits are preferably 2 ml/g and 20 ml/g, respectively.

The reaction temperature depends on the types of the solvent used and optically active compound used and represented by formula (4), but any temperature can be selected in the range from the freezing point to the boiling point of the reaction solvent used. The lower and upper limits of the reaction temperature are generally -20°C and 180°C, and preferably 30°C and 150°C, respectively.

Although the reaction time depends on the type of the optically active compound used and represented by formula (4), the reaction solvent, and the reaction temperature, the time is generally about 0.5 to 40 hours.

The degree of reaction progress can be determined by an ordinary analytical means, for example, time-lapse analysis of the reaction solution using high-performance liquid chromatography (HPLC). The completion of the reaction can be confirmed by the disappearance of the optically active compound represented by formula (4).

A post-treatment after the reaction is not particularly limited. However, after the completion of the reaction, for example, an aqueous diluted hydrochloric acid solution may be added to the reaction solution to terminate the reaction. Then, the target product may be extracted with an appropriate organic solvent such as ethyl acetate, toluene, benzene, diethyl ether, or dichloromethane. The extract may be washed with water and saturated brine and concentrated to isolate the target product. If required, the target product may be isolated and purified by, for example, column chromatography.

Finally, the third step will be described.

In this step, the optically active compound represented by formula (5) and prepared as described above is converted, by ring closure, to the optically active 1-substituted amino-2,3-epoxypropane represented by formula (6) which is a target compound in the present invention. In compound (5) used in this step, as described above, R⁵ represents a hydrogen atom or a formyl group, an acyl group, or an aroyl group, which is represented by COR³. In either case, the ring-closure reaction is performed in the presence of a base.

First, the optically active 1-substituted amino-2,3-epoxypropane represented by formula (6) is described. In compound (6), R¹ and R² are defined as described above. However, when compound (6) is used as an intermediate for preparing optically active pharmaceuticals, more preferably, one of R¹ and R² is a hydrogen atom, and the other is tert-butoxycarbonyl, benzyloxycarbonyl, or benzoyl, or R¹ and R² represent together a phthaloyl group. The 1-substituted amino-2,3-epoxypropane represented by formula (6) has an asymmetric carbon atom having configuration which is derived from that of the corresponding asymmetric carbon atom of the optically active compound represented by formula (5). In the step of ring-closure reaction, the configuration of the corresponding asymmetric carbon atom of the optically active compound represented by formula (5) is not changed and is inherited to the configuration of the asymmetric carbon atom of the optically active compound represented by formula (6).

Next, the step of ring-closure reaction will be described below.

When the optically active compound represented by formula (5) has group COR³ as R⁵, the base used is not particularly limited. However, a base having the ability to remove a formyl group, an acyl group, or an aroyl group substituting for a hydroxyl group of the optically compound represented formula (5) is used. Examples of the base include alkali metal hydroxides such as lithium hydroxide, sodium hydroxide, potassium hydroxide, and cesium hydroxide; alkaline earth metal hydroxide such as magnesium hydroxide, calcium hydroxide, and barium hydroxide; alkali metal alkoxides such as lithium methoxide, sodium methoxide, potassium ethoxide, and sodium isopropoxide; alkali metal hydrogen carbonates such as sodium hydrogen carbonate and potassium hydrogen carbonate; and alkali metal carbonates such as lithium carbonate, sodium carbonate, and potassium carbonate. Among these compounds, sodium hydroxide and potassium carbonate are preferred. When an alcohol solvent such as methanol; ethanol, propanol, or isopropanol is used as the reaction solvent, lithium, sodium, potassium, lithium hydride, or sodium hydride which react with such solvents to produce alkali metal alkoxides can also be used.

The lower and upper limits of the amount of the base used are 100 mol% and 500 mol%, and preferably 110 mol% and 300 mol%, respectively, relative to the optically active compound represented by formula (5), depending on the type of the base used.

In the above-described reaction, a solvent is generally used, and the solvent is not particularly limited unless the reaction is inhibited. Examples of the solvent include water; alcohol solvents such as methanol, ethanol, propanol, and isopropanol; ether solvents such as tetrahydrofuran and dioxane; N,N-dimethylformamide. These solvents may be used in combination of two or more. Among these solvents, alcohol solvents are preferred, and methanol is particularly preferred.

The reaction may be performed in a two-phase solvent system containing water and another solvent immiscible with water and unreactive to the base, for example, an aliphatic hydrocarbon solvent such as hexane or heptane, an aromatic hydrocarbon solvent such as benzene, toluene, or xylene, an ether solvent such as ether, methyl tert-butyl ether, or a halogenated hydrocarbon solvent such as methylene chloride, chloroform, carbon tetrachloride, or chlorobenzene. In this case, a phase-transfer catalyst is preferably added for increasing the reaction rate. Examples of the phase-transfer catalyst include quaternary ammonium salts such as aliquat 336, butylpyridinium bromide, benzyltriethylammonium bromide, benzyltriethylammonium chloride, benzyltrimethylammonium chloride, hexadecyltrimethylammonium bromide, dibutyldimethylammonium chloride, decyltriethylammonium bromide, methyltriphenylammonium bromide, octyltriethylammonium bromide, tetrabutylammonium bromide, tetrabutylammonium chloride, tetrabutylammonium iodide, tetraethylammonium chloride, tetramethylammonium bromide, and tetrabutylammonium hydrogensulfate; quaternary phosphonium salts such as hexadecyltriethylphosphonium bromide, hexadecyltributylphosphonium bromide, hexadecyltributylphosphonium chloride, tetrabutylphosphonium chloride, trioctylethylphosphonium bromide, and tetraphenylphosphonium bromide; crown ethers such as 18-crown-6, dibenzo-18-crown-6, and dicyclohexyl-18-crown-6. Among these compounds, quaternary ammonium salts are preferably used.

Although the amount of the solvent used is not particularly limited, the lower and upper limits of the amount are 1 ml/g and 100 ml/g, respectively, relative to the optically active compound represented by formula (5). In view of the reaction operationality and economics, the lower and upper limits are preferably 2 ml/g and 20 ml/g, respectively.

Also, the amount of the phase-transfer catalyst used is not particularly limited. However, a catalytic amount is generally used, and the lower limit and upper limit of the amount are 1 mol% and 100 mol%, respectively, relative to the optically active compound represented by formula (5). The phase-transfer catalyst can be used in any desired amount within this range.

The reaction temperature depends on the type of the solvent used, but any temperature can be selected in the range from the freezing point to the boiling point of the reaction solvent used. The lower and upper limits of the reaction temperature are generally -20°C and 150°C, and preferably 0° C and 100°C, respectively.

Although the reaction time depends on the type of the optically active compound used and represented by formula (5), the reaction solvent, and the reaction temperature, the time is generally about 0.5 to 24 hours.

The degree of reaction progress can be determined by an ordinary analytical means, for example, time-lapse analysis of the reaction solution by high-performance liquid chromatography (HPLC). The completion of the reaction can be confirmed by the disappearance of the optically active compound represented by formula (5).

A post-treatment after the reaction is not particularly limited. However, after the completion of the reaction, for example, a saturated aqueous ammonium chloride solution may be added to the reaction solution. Then, the target product may be extracted with an appropriate organic solvent such as ethyl acetate, toluene, benzene, diethyl ether, or dichloromethane. The extract may be washed with water and saturated brine or the like and concentrated to isolate the target product. When the reaction is preformed in the two-phase solvent system, the organic layer is separated from the aqueous layer and, if required, washed with water and then concentrated. If required, the target product may be purified by, for example, column chromatography to increase the purity.

When the optically active compound represented by formula (5) has a hydrogen atom as R⁵, the base used is not particularly limited. Examples of the base include alkali metal hydroxides such as lithium hydroxide, sodium hydroxide, potassium hydroxide, and cesium hydroxide; alkaline earth metal hydroxide such as magnesium hydroxide, calcium hydroxide, and barium hydroxide; alkali metal alkoxides such as lithium methoxide, sodium methoxide, potassium ethoxide, and sodium isopropoxide; alkali metal hydrogen carbonates such as sodium hydrogen carbonate and potassium hydrogen carbonate; and alkali metal carbonates such as lithium carbonate, sodium carbonate, and potassium carbonate; lithium; sodium; potassium; lithium hydride; and sodium hydride. Among these compounds, potassium carbonate and sodium methoxide are preferred.

The lower and upper limits of the amount of the base used are 100 mol% and 500 mol%, and preferably 110 mol% and 300 mol%, respectively, relative to the optically active compound represented by formula (5), depending on the type of the base used.

In the above-described reaction, a solvent is generally used, and the solvent is not particularly limited unless the reaction is inhibited. Although the solvent depends on the type of the base used, examples of the solvent include water; alcohol solvents such as methanol, ethanol, propanol, and isopropanol; ether solvents such as diethyl ether, diisopropyl ether, methyl tert-butyl ether, tetrahydrofuran and dioxane; aliphatic or alicyclic hydrocarbon solvents such as heptane, hexane, cyclohexane, methylcyclohexane; aromatic hydrocarbon solvents such as benzene, toluene, and xylene; ester solvents such as methyl acetate, ethyl acetate, and butyl acetate; ketone solvents such as acetone, ethyl methyl ketone; halogenated hydrocarbon solvents such as methylene chloride, 1,2-dichloroethane, chloroform, carbon tetrachloride, and chlorobenzene; nitrile solvents such as acetonitrile, propionitrile, and benzonitrile; N,N-dimethylformamide; and N,N-dimethylacetamide. These solvents may be used in combination of two or more. For example, when potassium carbonate is used as the base, alcohol solvents and ketone solvents are preferably used.

The reaction temperature depends on the type of the solvent used, but any temperature can be selected in the range from the freezing point to the boiling point of the reaction solvent used. The lower and upper limits of the reaction temperature are generally -20°C and 150°C, and preferably 0° C and 100°C, respectively.

Although the reaction time depends on the type of the optically active compound used and represented by formula (5), the reaction solvent, and the reaction temperature, the time is generally about 0.5 to 72 hours.

The degree of reaction progress can be determined by an ordinary analytical means, for example, time-lapse analysis of the reaction solution by high-performance liquid chromatography (HPLC). The completion of the reaction can be confirmed by the disappearance of the optically active compound represented by formula (5).

A post-treatment after the reaction is not particularly limited. However, after the completion of the reaction, for example, water may be added to the reaction solution to terminate the reaction. Then, the target product may be extracted with an appropriate organic solvent such as ethyl acetate, toluene, benzene, diethyl ether, or dichloromethane. The extract may be washed with water and saturated brine or the like and concentrated to isolate the target product. If required, the target product may be purified by, for example, column chromatography to increase the purity.

### Best Mode for Carrying Out the Invention

The present invention will be described in further detail below with reference to examples, but the present invention is not limited to these examples. In the examples, HPLC (high-performance liquid chromatographic) analysis was performed under the following conditions unless otherwise specified:
Column: Nacalai C8 4.6 mm I.D. × 250 mm
Mobile phase: 10 mM {NaH₂PO₄-Na₂HPO₄} aqueous solution (pH = 6.8)/acetonitrile = 1/1 (vol/vol)
Flow rate: 1 ml/min
Detection: UV 254 nm
Column temperature: 40° C
Injection volume: 10 µl

The optical purity was analyzed by high-performance liquid chromatography using an optically active analysis column under the following conditions unless otherwise specified:
Column: CHIRALCEL OD-H 4.6 mm I.D. × 250 mm
Mobile phase: n-hexane/isopropanol = 95/5 (vol/vol)
Flow rate: 0.5 ml/min
Detection: UV 254 nm
Column temperature:40° C
Injection volume: 10 µl

### (EXAMPLE 1) Preparation of (2R/S, 4S)-4-[(N-benzyloxycarbonyl)aminomethyl]-2-methoxy-1,3-dioxolane

### [Preparation of Compound (4)]

In 20 ml of toluene, 2.00 g (8.88 mmol) of (S)-1-(N-benzyloxycarbonyl)amino-2,3-propanediol was suspended, and 2.58 g (24.3 mmol, 270 mol%) of trimethyl orthoformate and 16 mg (0.267 mmol, 3.00 mol%) of acetic acid were added to the resultant suspension, followed by stirring at 110°C for 3 hours. After the mixture was cooled to room temperature, 10 ml of ammonia water was added to the mixture to terminate the reaction. After a separating operation, the aqueous layer was subjected to extraction with 10 ml of toluene two times, and the whole organic layer was washed with 10 ml of saturated brine and then dried over anhydrous magnesium sulfate. The organic layer was then concentrated under reduced pressure, and the residue was dried in vacuum to produce 2.47 g of (2R/S, 4S)-4-[(N-benzyloxycarbonyl)aminomethyl]-2-methoxy-1,3-dioxolane as a colorless oily diastereomer mixture.
HPLC retention time: 6.30 min., 6.61 min.
¹H NMR (400 MHz, CDCl₃) δ 7.36-7.33 (5H, m), 5.74, 5.70 (1H, s×2), 5.36, 5.14 (1H, bs×2), 5.14 (2H, s), 4.45-4.40, 4.35-4.30 (1H, m×2), 4.11-4.05, 4.16-4.12 (1H, m×2), 3.78-3.27 (3H, m), 3.32, 3.31 (3H, s×2)

### (EXAMPLE 2) Preparation of (2R/S, 4S)-4-[(N-benzyloxycarbonyl)aminomethyl]-2-ethoxy-1,3-dioxolane [Preparation of Compound (4)]

In 10 ml of toluene, 1.00 g (4.44 mmol) of (S)-1-(N-benzyloxycarbonyl)amino-2,3-propanediol was suspended, and 1.79 g (12.0 mmol, 270 mol%) of triethyl orthoformate and 7.9 mg (0.132 mmol, 2.98 mol%) of acetic acid were added to the resultant suspension, followed by stirring at 110°C for 3 hours. After the mixture was cooled to room temperature, 5 ml of ammonia water was added to the mixture to terminate the reaction. After a separating operation, the aqueous layer was subjected to extraction with 5 ml of toluene two times, and the whole organic layer was washed with 10 ml of saturated brine and then dried over anhydrous magnesium sulfate. The organic layer was then concentrated under reduced pressure, and the residue was dried in vacuum to produce 1.36 g of (2R/S, 4S)-4-[(N-benzyloxycarbonyl)aminomethyl]-2-ethoxy-1,3-dioxolane as a colorless oily diastereomer mixture.
HPLC retention time: 6.56 min., 7.00 min.
¹H NMR (400 MHz, CDCl₃) δ 7.36-7.30 (5H, m), 5.82, 5.78 (1H, s×2), 5.51, 5.20 (1H, bs×2), 5.17 (2H, s), 4.44-4.34, 4.34-4.33 (1H, m×2), 4.16-4.14, 4.01-4.04 (1H, m×2), 3.80-3.28 (3H, m), 3.62, 3.58 (2H, m×2), 1.23, 1.22 (3H, t×2, J=7.2Hz)

### (EXAMPLE 3) Preparation of (2R/S, 4S)-4-[(N-benzyloxycarbonyl)aminomethyl]-2-methoxy-2-methyl-1,3-dioxolane [Preparation of Compound (4)]

In 10 ml of methylene chloride, 2.00 g (8.88 mmol) of (S)-1-(N-benzyloxycarbonyl)amino-2,3-propanediol was dissolved, and 1.36 g (11.3 mmol, 130 mol%) of trimethyl orthoacetate and 4.3 mg (0.0226 mmol, 0.25 mol%) of p-toluenesulfonic acid monohydrate were added to the resultant solution, followed by stirring at room temperature for 1 hour. After the solvent was distilled off under reduced pressure, the residue was dried in vacuum to produce 2.48 g of (2R/S, 4S)-4-[(N-benzyloxycarbonyl)aminomethyl]-2-methoxy-2-methyl-1,3-dioxolane as a colorless oily diastereomer mixture.
HPLC retention time: 6.78 min., 7.15 min.
¹H NMR (400 MHz, CDCl₃) δ 7.36-7.30 (5H, m), 5.38, 5.08 (1H, bs×2), 5.11 (2H, s), 4.41-4.40, 4.39-4.32 (1H, m×2), 4.16, 4.09 (1H, t×2, J=7.6Hz), 3.72, 3.67 (1H, t×2, J=7.6Hz), 3.55-3.44 (1H, m), 3.39-3.24 (1H, m), 3.27 (s, 3H), 1.56, 1.55 (3H, s×2)

### (EXAMPLE 4) Preparation of (2R/S, 4S)-4-[(N-benzyloxycarbonyl)aminomethyl]-2-ethoxy-2-methyl-1,3-dioxolane [Preparation of Compound (4)]

In 5 ml of methylene chloride, 1.00 g (4.44 mmol) of (S)-1-(N-benzyloxycarbonyl)amino-2,3-propanediol was dissolved, and 0.927 g (5.68 mmol, 130 mol%) of triethyl orthoacetate and 2.3 mg (0.0120 mmol, 0.27 mol%) of p-toluenesulfonic acid monohydrate were added to the resultant solution, followed by stirring at room temperature for 1 hour. After the solvent was distilled off under reduced pressure, the residue was dried in vacuum to produce 1.34 g of (2R/S, 4S)-4-[(N-benzyloxycarbonyl)aminomethyl]-2-ethoxy-2-methyl-1,3-dioxolane as a colorless oily diastereomer mixture.
HPLC retention time: 7.52 min., 8.00 min.
¹H NMR (400 MHz, CDCl₃) δ 7.37-7.31 (5H, m), 5.58, 5.11 (1H, bs×2), 5.11 (2H, s), 4.42-4.32 (1H, m), 4.15, 4.08 (1H, t×2, J=7.6Hz), 3.76, 3.70 (1H, t×2, J=7.6Hz), 3.54, 3.53 (2H, q×2, J=7.1Hz), 3.39, 3.35 (1H, t×2, J=5.8Hz), 3.31, 3.26 (1H, t×2, J=6.1Hz), 1.57, 1.56 (3H, s×2), 1.18, 1.17 (3H, t×2, J=7.1Hz)

### (EXAMPLE 5) Preparation of (2R/S, 4S)-4-[(N-benzyloxycarbonyl)aminomethyl]-2-methoxy-2-propyl-1,3-dioxolane [Preparation of Compound (4)]

In 5 ml of methylene chloride, 1.00 g (4.44 mmol) of (S)-1-(N-benzyloxycarbonyl)amino-2,3-propanediol was dissolved, and 0.844 g (5.68 mmol, 130 mol%) of trimethyl orthobutylate and 2.1 mg (0.0110 mmol, 0.25 mol%) of p-toluenesulfonic acid monohydrate were added to the resultant solution, followed by stirring at room temperature for 1 hour. After the solvent was distilled off under reduced pressure, the residue was dried in vacuum to produce 1.38 g of (2R/S, 4S)-4-[(N-benzyloxycarbonyl)aminomethyl]-2-methoxy-2-propyl-1,3-dioxolane as a colorless oily diastereomer mixture.
HPLC retention time: 21.6 min., 24.5 min.
¹H NMR (400 MHz, CDCl₃) δ 7.37-7.30 (5H, m), 5.38, 5.05 (1H, bs×2), 5.11 (2H, s), 4.42-4.39, 4.39-4.29 (1H, m×2), 4.17, 4.08 (1H, t×2, J=8.0Hz), 3.72, 3.67 (1H, t×2, J=8.0Hz), 3.36-3.45 (1H, m), 3.72, 3.32 (1H, t×2, J=6.0Hz), 3.25 (3H, s), 1.78, 1.75 (2H, t×2, J=3.6Hz), 1.46, 1.43 (2H, m×2), 0.934, 0.927 (3H, t×2, J=3.6Hz)

### (EXAMPLE 6) Preparation of (2R/S, 4S)-4-[(N-benzyloxycarbonyl)aminomethyl]-2-methoxy-2-phenyl-1,3-dioxolane [Preparation of Compound (4)]

In 5 ml of methylene chloride, 1.00 g (4.44 mmol) of (S)-1-(N-benzyloxycarbonyl)amino-2,3-propanediol was dissolved, and 1.05 g (5.68 mmol, 130 mol%) of trimethyl orthobenzoate and 2.63 mg (0.0138 mmol, 0.31 mol%) of p-toluenesulfonic acid monohydrate were added'to the resultant solution, followed by stirring at room temperature for 1 hour. After the solvent was distilled off under reduced pressure, the residue was dried in vacuum to produce 1.38 g of (2R/S, 4S)-4-[(N-benzyloxycarbonyl)aminomethyl]-2-methoxy-2-phenyl-1,3-dioxolane as a colorless oily diastereomer mixture.
HPLC retention time: 20.6 min., 23.8 min.
¹H NMR (400 MHz, CDCl₃) δ 7.58-7.54 (2H, m), 7.44-7.34 (8H, m), 5.45, 4.96 (1H, bs×2), 5.13, 5.09 (2H, s×2), 4.58-4.59, 4.39-4.30 (1H, m×2), 4.32, 4.22 (1H, t×2, J=7.6Hz), 3.89-3.86 (1H, m), 3.50-3.47 (1H, m), 3.35-3.29 (1H, m), 3.21, 3.20 (3H, s×2)

### (EXAMPLE 7) Preparation of (2R/S, 4S)-4-[(N-tert-butoxycarbonyl)aminomethyl]-2-methoxy-2-methyl-1,3-dioxolane

### [Preparation of Compound (4)]

In 10 ml of toluene, 1.51 g (7.92 mmol) of (S)-1-(N-tert-butoxycarbonyl)amino-2,3-propanediol was dissolved, and 2.56 g (21.4 mmol, 270 mol%) of trimethyl orthoacetate and 12 mg (0.20 mmol, 2.53 mol%) of acetic acid were added to the resultant solutiori, followed by stirring at room temperature for 5 hours and at 50°C for 24 hours. Since the raw materials did not disappear, 10 mg (0.17 mmol, 2.10 mol%) of acetic acid was further added to the mixture, followed by stirring for 18 hours. After the disappearance of the raw materials was confirmed, the mixture was cooled to room temperature, and the solvent was distilled off under reduced pressure. The residue was dried in vacuum to produce 1.92 g of (2R/S, 4S)-4-[(N-tert-butoxycarbonyl)aminomethyl]-2-methoxy-2-methyl-1,3-dioxolane as a colorless oily diastereomer mixture.
HPLC analytical condition; column: Nacalai C8 4.6 mm I.D. × 250 mm, mobile phase: 10 mM {NaH₂PO₄-Na₂HPO₄} aqueous solution (pH = 6.8)/acetonitrile = 1/1(vol/vol), flow rate: 1 ml/min, detection: UV 210 nm, column temperature: 40°C, Injection volume: 10 µl
HPLC retention time: 6.0 min., 6.3 min.
¹H NMR (400 MHz, CDCl₃) δ 5.12, 4.83 (1H, bs×2), 4.39-4.37, 4.37-4.29 (1H, m×2), 4.15, 4.08 (1H, t×2, J=8.0Hz), 3.74-3.68 (1H, m), 3.50-3.47 (1H, m), 3.29, 3.28 (3H, s×2), 3.26-3.19 (1H, m×2), 1.57, 1.55 (3H, s×2), 1.45 (9H,s)

### (EXAMPLE 8) Preparation of (2R/S, 4S)-4-[(N-benzyloxycarbonyl)aminomethyl]-2-methoxy-2-methyl-1,3-dioxolane [Preparation of Compound (4)]

In 4 ml of toluene, 202 mg (0.888 mmol) of (S)-1-(N-benzyloxycarbonyl)amino-2,3-propanediol was suspended, and 298 mg (2.48 mmol, 280 mol%) of trimethyl orthoacetate and 9.4 mg (0.0690 mmol, 7.77 mol%) of zinc chloride were added to the resultant suspension, followed by stirring at 110° C for 2 hours. After the resultant mixture was cooled to room temperature, 5 ml of water was added to the mixture to terminate the reaction. After a separating operation, the aqueous layer was subjected to two times of extractions with 10 ml of toluene each and then concentration under reduced pressure. The residue was dried in vacuum to produce 239 mg of (2R/S, 4S)-4-[(N-benzyloxycarbonyl)aminomethyl]-2-methoxy-2-methyl-1,3-dioxolane as a colorless oily diastereomer mixture.

### (EXAMPLE 9) Preparation of (2R/S, 4S)-4-[(N-benzyloxycarbonyl)aminomethyl]-2-methoxy-2-methyl-1,3-dioxolane [Preparation of Compound (4)]

In 3 ml of toluene, 200 mg (0.888 mmol) of (S)-1-(N-benzyloxycarbonyl)amino-2,3-propanediol was suspended, and 298 mg (2.48 mmol, 280 mol%) of trimethyl orthoacetate and 10 mg (0.0328 mmol, 3.70 mol%) of triethylamine sulfate were added to the resultant suspension, followed by stirring at 110°C for 2 hours. After the resultant mixture was cooled to room temperature, 5 ml of water was added to the mixture to terminate the reaction. After a separating operation, the aqueous layer was subjected to two times of extractions with 10 ml of toluene each and then concentration under reduced pressure. The residue was dried in vacuum to produce 253 mg of (2R/S, 4S)-4-[(N-benzyloxycarbonyl)aminomethyl]-2-methoxy-2-methyl-1,3-dioxolane as a colorless oily diastereomer mixture.

### (EXAMPLE 10) Preparation of (2R/S, 4S)-4-[(N-benzyloxycarbonyl)aminomethyl]-2-methoxy-2-methyl-1,3-dioxolane [Preparation of Compound (4)]

In 3 ml of toluene, 200 mg (0.888 mmol) of (S)-1-(N-benzyloxycarbonyl)amino-2,3-propanediol was suspended, and 300 mg (2.50 mmol, 280 mol%) of trimethyl orthoacetate and silica gel (9.4 mg) were added to the resultant suspension, followed by stirring at 110°C for 2 hours. As a result of the analysis of the reaction solution by high-performance liquid chromatography, it was confirmed that the raw materials remained, but (2R/S, 4S)-4-[(N-benzyloxycarbonyl)aminomethyl]-2-methoxy-2-methyl-1,3-dioxolane was produced as a main product.

### (EXAMPLE 11) Preparation of (2R/S, 4R)-4-[(N-benzyloxycarbonyl)aminomethyl]-2-methoxy-2-methyl-1,3-dioxolane [Preparation of Compound (4)]

In 450 ml of toluene, 46.5 g (purity: 96.7 wt%, 0.200 mol) of (R)-1-(N-benzyloxycarbonyl)amino-2,3-propanediol was suspended, and 36.0 g (0.299 mol, 150 mol%) of trimethyl orthoacetate and 360 mg (5.99 mmol, 3.00 mol%) of acetic acid were added to the resultant suspension, followed by stirring at 110°C for 3 hours. As a result of analysis by high-performance liquid chromatography, it was confirmed that (2R/S, 4R)-4-[(N-benzyloxycarbonyl)aminomethyl]-2-methoxy-2-methyl-1,3-dioxolane was produced.
HPLC retention time: 6.30 min., 6.61min.

### (EXAMPLE 12) Preparation of (2R/S, 4S)-4-[(N-benzyloxycarbonyl)aminomethyl]-1,3,2λ⁴-dioxathiolane-2-one

### [Preparation of Compound (4)]

In 40 ml of chloroform, 2.068 g (9.18 mmol) of (S)-1-(N-benzyloxycarbonyl)amino-2,3-propanediol was dissolved, and 2.85 g (23.8 mmol, 260 mol%) of thionyl chloride was added dropwise to the resultant solution under ice-cooling over 10 minutes. Furthermore, 3.72 g (36.7 mmol, 400 mol%) of triethylamine was added dropwise to the resultant mixture over 15 minutes. After the addition, the mixture was stirred at room temperature for 1 hour and then concentrated under reduced pressure. Then, 10 ml of water was added to the mixture to terminate the reaction, and the reaction solution was subjected to two times of extractions with 30 ml of diethyl ether each. The whole organic layer was washed with 30 ml of 2N hydrochloric acid twice and 30 ml of saturated brine, and dried over anhydrous magnesium sulfate. The organic layer was concentrated under reduced pressure, and the residue was dried in vacuum to produce 2.22 g of (2R/S, 4S)-4-[(N-benzyloxycarbonyl)aminomethyl]-1,3,2λ⁴-dioxathiolane-2-one as a reddish-brown oily diastereomer mixture (yield 89.2%).
HPLC retention time: 17.40 min., 18.8 min.
¹H NMR (400 MHz, CDCl₃) δ 7.37-7.31 (5H, m), 5.37, 5.07 (1H, bs×2), 5.13 (2H, s), 5.02-5.08, 4.22-4.19 (2H, m×2), 4.74-4.70, 3.67-3.65 (2H, m×2), 4.53-4.41, 3.58-3.41 (1H, m×2)

### (EXAMPLE 13) Preparation of (2R/S, 4S)-4-[(N-benzyloxycarbonyl)aminomethyl]-1,3,2λ⁴-dioxathiolane-2-one

### [Preparation of Compound (4)]

In 5 ml of toluene, 334.1 mg (1.48 mmol) of (S)-1-(N-benzyloxycarbonyl)amino-2,3-propanediol was suspended, and 0.25 ml (3.43 mmol, 231 mol%) of thionyl chloride was added to the resultant suspension under ice-cooling at a time. Furthermore, 0.9 ml (6.46 mmol, 435 mol%) of triethylamine was added dropwise to the resultant mixture over 5 minutes. After the addition, the mixture was stirred under ice-cooling for 140 minutes and at room temperature for 35 minutes. After the reaction mixture was again cooled with ice, 8 ml of toluene and 6 ml of cold water were added to the reaction mixture. After the organic layer was separated, the aqueous layer was subjected to two times of extractions with 5 ml of toluene each. The whole organic layer was dried over anhydrous sodium sulfate, and then concentrated under reduced pressure. The resulting oily residue was purified by silica gel column chromatography (eluent: toluene → diethyl ether/hexane = 1/5 (ratio by volume hereinafter) → diethyl ether/hexane = 2/1) to produce 369.5 mg of (2R/S, 4S)-4-[(N-benzyloxycarbonyl)aminomethyl]-1,3,2λ⁴-dioxathiolane-2-one as an orangish-yellow oily diastereomer mixture (yield 91.8%).

### (EXAMPLE 14) Preparation of (2R/S, 4S)-4-[(N-benzyloxycarbonyl)aminomethyl]-1,3,2λ⁴-dioxathiolane-2-one

### [Preparation of Compound (4)]

In 4 ml of toluene, 331.6 mg (1.47 mmol) of (S)-1-(N-benzyloxycarbonyl)amino-2,3-propanediol, 209.4 mg (1.9 mmol, 129 mol%) of dimethyl sulfite, and 1.6 mg (0.017 mmol, 1.13 mol%) of methanesulfonic acid were suspended, and the resultant suspension was refluxed for 130 minutes. Since thin-layer chromatography of the reaction mixture showed a large amount of the remaining diol used as the raw material, 556.1 mg (5.05 mmol, 343 mol%, 472 mol% in total) of dimethyl sulfite was added, and the resultant mixture was further refluxed for 7 hours. During the reflux, a low-boiling-point distillate was removed. After the reaction solution was cooled to room temperature, the reaction solution was neutralized with three droplets of triethylamine and then purified by silica gel column chromatography (eluent: toluene → diethyl ether/hexane = 1/5 → diethyl ether/hexane = 2/1 → diethyl ether → ethyl acetate) to produce 263 mg of (2R/S,4S)-4-[(N-benzyloxycarbonyl)aminomethyl]-1,3,2λ⁴-dioxathiolane-2-one as a colorless oily diastereomer mixture (yield 65.8%). Also, 94.7 mg of (S)-1-(N-benzyloxycarbonyl)amino-2,3-propanediol used as a raw material was recovered as white crystals (yield 28.6%).

### (EXAMPLE 15) Preparation of (2R/S,4R)-4-[(N-benzyloxycarbonyl)aminomethyl]-1,3,2λ⁴-dioxathiolane-2-one

### [Preparation of Compound (4)]

In 20 ml of methylene chloride, 1078.7 mg (4.79 mmol) of (R)-1-(N-benzyloxycarbonyl)amino-2,3-propanediol was suspended, and 0.9 ml (12.34 mmol, 258 mol%) of thionyl chloride was added to the resultant suspension under ice cooling at a time. Furthermore, 3.4 ml (24.39 mmol, 509 mol%) of triethylamine was added dropwise to the resulting mixture over 5 minutes. After the addition, the mixture was stirred under ice cooling for 90 minutes and at room temperature for 30 minutes. Then, the reaction mixture was poured into 30 ml of water containing 2 ml of 4N hydrochloric acid, and 10 ml of methylene chloride was added for extraction. After a separating operation, the aqueous layer was subjected to further extraction with 20 ml of methylene chloride. The whole organic layer was dried over anhydrous sodium sulfate and concentrated under reduced pressure. The resulting oily substance was purified by silica gel column chromatography (eluent: diethyl ether/hexane = 1/5 → methylene chloride/hexane = 1/5 → diethyl ether/hexane = 1/5 → diethyl ether/hexane = 1/2 → diethyl ether/hexane = 2/1) to produce 1271.6 mg of (2R/S, 4R)-4-[(N-benzyloxycarbonyl)aminomethyl]-1,3,2λ⁴-dioxathiolane-2-one as an orangish-yellow oily diastereomer mixture (yield 97.9%).
¹H NMR (400MHz, CDCl₃) δ 7.37-7.31 (5H, m), 5.37, 5.07 (1H, bs×2), 5.13 (2H, s), 5.02-5.08, 4.22-4.19 (2H, m×2), 4.74-4.70, 3.67-3.65 (2H, m×2), 4.53-4.41, 3.58-3.41 (1H, m×2)

### (EXAMPLE 16) Preparation of (2R/S, 4S)-4-(N-benzoylaminomethyl)-1,3,2λ⁴-dioxathiolane-2-one [Preparation of Compound (4)]

In 15 ml of methylene chloride, 420 mg (2.15 mmol) of (S)-1-(N-benzoylamino)-2,3-propanediol was suspended, and 0.42 ml (5.76 mmol, 268 mol%) of thionyl chloride was added to the resultant'suspension under ice cooling at a time. Furthermore, 1.8 ml (12.91 mmol, 600 mol%) of triethylamine was added dropwise to the resulting mixture over 5 minutes. After the addition, the mixture was stirred under ice cooling for 75 minutes. Then, 20 ml of water containing 1.5 ml of 4N hydrochloric acid was added to the reaction mixture, and 5 ml of methylene chloride was added for extraction. After a separating operation, the aqueous layer was subjected to further extraction with 15 ml of methylene chloride. The whole organic layer was dried over anhydrous sodium sulfate and concentrated under reduced pressure. The resulting oily substance was purified by silica gel column chromatography (eluent: methylene chloride/hexane = 1/2 → methylene chloride/hexane = 1/1 → diethyl ether/hexane = 1/3 → diethyl ether/hexane = 1/2 → diethyl ether/hexane = 1/1 →diethyl ether/hexane = 2/1 → diethyl ether) to produce 417.1 mg of (2R/S, 4S)-4-(N-benzoylaminomethyl)-1,3,2λ⁴-dioxathiolane-2-one as an orangish-yellow solid diastereomer mixture (yield 80.4%).
¹H NMR (400 MHz, CDCl₃) δ 7.89-7.75 (2H, m), 7.57-7.41 (3H, m), 6.97, 6.54 (1H, bs×2), 5.21-5.14, 4.97-4.89 (1H, m×2), 4.81-4.75, 4.60-4.54, 4.53-4.46, 4.31-4.25 (2H, m×4), 4.20-4.11, 3.86-3.70 (2H, m×2)

### (EXAMPLE 17) Preparation of (2R/S, 4R)-4-phthalimidomethyl-1,3,2λ⁴-dioxathiolane-2-one [Preparation of Compound (4)]

In 55 ml of dichloroethane, 2.57 g (11.62 mmol) of (R)-1-phthalimido-2,3-propanediol was suspended, and 4.05 g (34.04 mmol, 293 mol%) of thionyl chloride was added to the resultant suspension under ice-cooling over 2 minutes. The resultant mixture was stirred at the same temperature for 15 minutes and at room temperature for 20 minutes. Then, 5.6 ml (69.24 mmol, 596 mol%) of pyridine was added dropwise to the mixture under ice-cooling over 2 minutes. After the addition, the dropping funnel used for adding pyridine was washed with 5 ml of dichloroethane, and the dichloroethane was added to the mixture, followed by stirring at the same temperature for 1 hour and at room temperature for 15 hours. Then, 50 ml of water containing 0.5 ml of concentrated hydrochloric acid was added to the reaction mixture at room temperature, and the mixture was subjected to extraction. After a separating operation, the aqueous layer was further subjected to two times of extractions with 15 ml of methylene chloride each. The whole organic layer was concentrated under reduced pressure without being dried, and then dried in vacuum to produce 3.41 g of (2R/S, 4R)-4-phthalimidomethyl-1,3,2λ⁴-dioxathiolane-2-one as a lightly yellowish white solid diastereomer mixture (quantitative yield).
¹H NMR (400 MHz, CDCl₃) δ 7.91-7.85 (2H, m), 7.79-7.73 (2H, m), 5.26-5.18, 4.43-4.38 (1H, m×2), 4.83-4.73 (1H, m), 4.68-4.57 (1H, m), 4.26-4.11 (1H, m), 4.02-3.83 (1H, m)

### (EXAMPLE 18) Preparation of (S)-2-(N-benzyloxycarbonyl)amino-1-(chloromethyl)ethyl formate

### [Preparation of Compound (5)]

In 5 ml of methylene chloride, 511.4 mg (1.82 mmol) of (2R/S, 4S)-4-[(N-benzyloxycarbonyl)aminomethyl]-2-ethoxy-1,3-dioxolane prepared in EXAMPLE 2 was dissolved, and 5 ml of a methylene chloride solution containing 470.5 mg (2.29 mmol, 120 mol%) of phosphorus pentachloride was added to the resultant solution under ice-cooling over 5 minutes. After the addition, the mixture was warmed to room temperature and then stirred for 5 hours. Then, 10 ml of saturated aqueous sodium bicarbonate was added to the mixture to terminate the reaction, and extraction with 10 ml of ethyl acetate was performed three times. The whole organic layer was washed with 30 ml of saturated aqueous sodium bicarbonate three times, 30 ml of water twice, and 30 ml of saturated brine once, and then dried over anhydrous magnesium sulfate. The organic layer was then concentrated under reduced pressure and dried in vacuum to produce 392 mg of (S)-2-(N-benzyloxycarbonyl)amino-1-(chloromethyl)ethyl formate as a colorless oily compound. The overall yield from (S)-1-(N-benzyloxycarbonyl)amino-2,3-propanediol was 69%. HPLC retention time: 7.2 min.
¹H NMR (400 MHz, CDCl₃) δ 8.09 (1H, s), 7.40-7.29 (5H, m), 5.34-5.33 (1H, m), 5.11 (2H, s), 4.98 (1H, bs), 3.75-3.50 (4H, m)

### (EXAMPLE 19) Preparation of (S)-2-(N-benzyloxycarbonyl)amino-1-(chloromethyl)ethyl acetate

### [Preparation of Compound (5)]

In 5 ml of methylene chloride, 508 mg (1.72 mmol) of (2R/S,4S)-4-[(N-benzyloxycarbonyl)aminomethyl]-2-ethoxy-2-methyl-1,3-dioxolane prepared in EXAMPLE 4 was dissolved, and 266 mg (2.31 mmol, 130 mol%) of trimethylsilyl chloride was added dropwise to the resultant solution over 5 minutes. After the addition, the mixture was stirred at room temperature for 2 hours. Then, 10 ml of saturated aqueous sodium bicarbonate was added to the mixture to terminate the reaction, and extraction with 10 ml of ethyl acetate was performed three times. The whole organic layer was washed with 30 ml of saturated brine once and then dried over anhydrous magnesium sulfate. The organic layer was then concentrated under reduced pressure and dried in vacuum to produce 440 mg of a crude product. The crude product was purified by preparative thin-layer chromatography (developing solvent; ethyl acetate/n-hexane= 1/2) to produce 387 mg of (S)-2-(N-benzyloxycarbonyl)amino-1-(chloromethyl)ethyl acetate as a colorless oily compound. The overall yield from (S)-1-(N-benzyloxycarbonyl)amino-2,3-propanediol was 82%.
HPLC retention time: 8.9 min.
¹H NMR (400 MHz, CDCl₃) δ 7.36-7.31 (5H, m), 5.14 (2H, s), 5.14-5.01 (1H, m), 4.96 (1H, bs), 3.71-3.46 (4H, m), 2.08 (3H, s)

### (EXAMPLE 20) Preparation of (S)-2-(N-benzyloxycarbonyl)amino-1-(chloromethyl)ethyl butylate

### [Preparation of Compound (5)]

In 5 ml of methylene chloride, 503 mg (1.62 mmol) of (2R/S, 4S)-4-[(N-benzyloxycarbonyl)aminomethyl]-2-methoxy-2-propyl-1,3-dioxolane prepared in EXAMPLE 5 was dissolved, and 221 mg (2.03 mmol, 130 mol%) of trimethylsilyl chloride was added dropwise to the resultant solution over 5 minutes. After the addition, the mixture was stirred at room temperature for 14 hours. Then, 10 ml of saturated aqueous sodium bicarbonate was added to the mixture to terminate the reaction, and extraction with 10 ml of ethyl acetate was performed three times. The whole organic layer was washed with 30 ml of saturated brine once and then dried over anhydrous magnesium sulfate. The organic layer was then concentrated under reduced pressure and dried in vacuum to produce 432 mg of a crude product. The crude product was purified by preparative thin-layer chromatography (developing solvent; ethyl acetate/n-hexane= 1/2) to produce 390 mg of (S)-2-(N-benzyloxycarbonyl)amino-1-(chloromethyl)ethyl butylate as a colorless oily compound. The overall yield from (S)-1-(N-benzyloxycarbonyl)amino-2,3-propanediol was 73%.
HPLC retention time: 13.7 min.
¹H NMR (400 MHz, CDCl₃) δ 7.36-7.31 (5H, m), 5.10 (2H, s), 5.10-5.00 (1H, m), 4.96 (1H, bs), 3.70-3.49 (4H, m), 2.31 (2H, J=7.2Hz, t), 1.65 (2H, m), 0.95 (3H, J=7.2Hz, t)

### (EXAMPLE 21) Preparation of (S)-2-(N-benzyloxycarbonyl)amino-1-(chloromethyl)ethyl benzoate

### [Preparation of Compound (5)]

In 5 ml of methylene chloride, 507 mg (1.54 mmol) of (2R/S, 4S)-4-[(N-benzyloxycarbonyl)aminomethyl]-2-methoxy-2-phenyl-1,3-dioxolane prepared in EXAMPLE 6 was dissolved, and 215 mg (1.98 mmol, 130 mol%) of trimethylsilyl chloride was added dropwise to the resultant solution over 5 minutes. After the addition, the mixture was stirred at room temperature for 2 hours. Then, 10 ml of saturated aqueous sodium bicarbonate was added to the mixture to terminate the reaction, and extraction with 10 ml of ethyl acetate was performed three times. The whole organic layer was washed with 30 ml of saturated brine once and then dried over anhydrous magnesium sulfate. The organic layer was then concentrated under reduced pressure and dried in vacuum to produce 468 mg of a crude product. The crude product was purified by preparative thin-layer chromatography (developing solvent; ethyl acetate/n-hexane= 1/2) to produce 303 mg of (S)-2-(N-benzyloxycarbonyl)amino-1-(chloromethyl)ethyl benzoate as a colorless oily compound. The overall yield from (S)-1-(N-benzyloxycarbonyl)amino-2,3-propanediol was 47%.
HPLC retention time: 7.3 min.
¹H NMR (400 MHz, CDCl₃) δ 8.05 (2H, d, J=7.2Hz), 7.56 (1H, t, J=7.6Hz), 7.45 (2H, t, J=7.6Hz), 7.36-7.31 (5H, m), 5.36-5.33 (1H, m), 5.09 (2H, s), 5.03 (1H, bs), 3.84-3.74 (2H, m), 3.69-3.63 (2H, m)

### (EXAMPLE 22) Preparation of (S)-2-(N-benzyloxycarbonyl)amino-1-(chloromethyl)ethyl acetate

### [Preparation of Compound (5)]

In 3 ml of methylene chloride, 200 mg (0.711 mmol) of (2R/S, 4S)-4-[(N-benzyloxycarbonyl)aminomethyl]-2-methoxy-2-methyl-1,3-dioxolane prepared by the same method as in EXAMPLE 3 was dissolved, and 110 mg (0.924 mmol, 130 mol%) of thionyl chloride was added to the resultant solution. After the addition, the mixture was stirred at room temperature for 2 hours. Then, 5 ml of saturated aqueous sodium bicarbonate was added to the mixture to terminate the reaction, and extraction with 10 ml of ethyl acetate was performed twice. The whole organic layer was washed with 30 ml of saturated brine once. As a result of analysis of the organic layer by high-performance liquid chromatography, it was confirmed that (S)-2-(N-benzyloxycarbonyl)amino-1-(chloromethyl)ethyl acetate was produced from (S)-1-(N-benzyloxycarbonyl)amino-2,3-propanediol in an overall yield of 92%.

### (EXAMPLE 23) Preparation of (S)-2-(N-benzyloxycarbonyl)amino-1-(chloromethyl)ethyl acetate

### [Preparation of Compound (5)]

In 3 ml of methylene chloride, 214 mg (0.763 mmol) of (2R/S, 4S)-4-[(N-benzyloxycarbonyl)aminomethyl]-2-methoxy-2-methyl-1,3-dioxolane prepared by the same method as in EXAMPLE 3 was dissolved, and 115 mg (1.01 mmol, 130 mol%) of sulfuryl chloride was added to the resultant solution. After the addition, the mixture was stirred at room temperature for 2 hours. Then, 5 ml of saturated aqueous sodium bicarbonate was added to the mixture to terminate the reaction, and extraction with 10 ml of ethyl acetate was performed twice. The whole organic layer was washed with 30 ml of saturated brine once. As a result of analysis of the organic layer by high-performance liquid chromatography, it was confirmed that (S)-2-(N-benzyloxycarbonyl)amino-1-(chloromethyl)ethyl acetate was produced from (S)-1-(N-benzyloxycarbonyl)amino-2,3-propanediol in an overall yield of 56%.

### (EXAMPLE 24) Preparation of (S)-2-(N-benzyloxycarbonyl)amino-1-(chloromethyl)ethyl acetate

### [Preparation of Compound (5)]

In 3 ml of methylene chloride, 204 mg (0.711 mmol) of (2R/S, 4S)-4-[(N-benzyloxycarbonyl)aminomethyl]-2-methoxy-2-methyl-1,3-dioxolane prepared by the same method as in EXAMPLE 3 was dissolved, and 142 mg (0.924 mmol, 130 mol%) of phosphorus oxychloride was added to the resultant solution. After the addition, the mixture was stirred at room temperature for 2 hours. Then, 5 ml of saturated aqueous sodium bicarbonate was added to the mixture to terminate the reaction, and extraction with 10 ml of ethyl acetate was performed twice. The whole organic layer was washed with 30 ml of saturated brine once. As a result of analysis of the organic layer by high-performance liquid chromatography, it was confirmed that (S)-2-(N-benzyloxycarbonyl)amino-1-(chloromethyl)ethyl acetate was produced from (S)-1-(N-benzyloxycarbonyl)amino-2,3-propanediol in an overall yield of 86%.

### (EXAMPLE 25) Preparation of (S)-2-(N-benzyloxycarbonyl)amino-1-(chloromethyl)ethyl acetate

### [Preparation of Compound (5)]

In 3 ml of methylene chloride, 199 mg (0.711 mmol) of (2R/S, 4S)-4-[(N-benzyloxycarbonyl)aminomethyl]-2-methoxy-2-methyl-1,3-dioxolane prepared by the same method as in EXAMPLE 3 was dissolved, and 74.3 mg (0.924 mmol, 130 mol%) of acetyl chloride was added to the resultant solution. After the addition, the mixture was stirred at room temperature for 2 hours. Then, 5 ml of saturated aqueous sodium bicarbonate was added to the mixture to terminate the reaction, and extraction with 10 ml of ethyl acetate was performed twice. The whole organic layer was washed with 30 ml of saturated brine once. As a result of analysis of the organic layer by high-performance liquid chromatography, it was confirmed that (S)-2-(N-benzyloxycarbonyl)amino-1-(chloromethyl)ethyl acetate was produced from (S)-1-(N-benzyloxycarbonyl)amino-2,3-propanediol in an overall yield of 72%.

### (EXAMPLE 26) Preparation of (S)-2-(N-benzyloxycarbonyl)amino-1-(chloromethyl)ethyl acetate

### [Preparation of Compound (5)]

In 3 ml of toluene, 200 mg (0.711 mmol) of (2R/S, 4S)-4-[(N-benzyloxycarbonyl)aminomethyl]-2-methoxy-2-methyl-1,3-dioxolane prepared by the same method as in EXAMPLE 3 was dissolved, and 100 mg (0.924 mmol, 130 mol%) of trimethylsilyl chloride was added to the resultant solution. After the addition, the mixture was stirred at room temperature for 2 hours. Then, 5 ml of saturated aqueous sodium bicarbonate was added to the mixture to terminate the reaction, and extraction with 10 ml of ethyl acetate was performed twice. The whole organic layer was washed with 30 ml of saturated brine once. As a result of analysis of the organic layer by high-performance liquid chromatography, it was confirmed that (S)-2-(N-benzyloxycarbonyl)amino-1-(chloromethyl)ethyl acetate was produced from (S)-1-(N-benzyloxycarbonyl)amino-2,3-propanediol in an overall yield of 87%.

### (EXAMPLE 27) Preparation of (S)-2-(N-benzyloxycarbonyl)amino-1-(chloromethyl)ethyl acetate

### [Preparation of Compound (5)]

In 3 ml of acetonitrile, 199 mg (0.711 mmol) of (2R/S, 4S)-4-[(N-benzyloxycarbonyl)aminomethyl]-2-methoxy-2-methyl-1,3-dioxolane prepared by the same method as in EXAMPLE 3 was dissolved, and 109 mg (0.924 mmol, 130 mol%) of trimethylsilyl chloride was added to the resultant solution. After the addition, the mixture was stirred at room temperature for 2 hours. Then, 5 ml of saturated aqueous sodium bicarbonate was added to the mixture to terminate the reaction, and extraction with 10 ml of ethyl acetate was performed twice. The whole organic layer was washed with 30 ml of saturated brine once. As a result of analysis of the organic layer by high-performance liquid chromatography, it was confirmed that (S)-2-(N-benzyloxycarbonyl)amino-1-(chloromethyl)ethyl acetate was produced from (S)-1-(N-benzyloxycarbonyl)amino-2,3-propanediol in an overall yield of 85%.

### (EXAMPLE 28) Preparation of (S)-2-(N-benzyloxycarbonyl)amino-1-(bromomethyl)ethyl acetate

### [Preparation of Compound (5)]

In 5 ml of methylene chloride, 200 mg (0.711 mmol) of (2R/S, 4S)-4-[(N-benzyloxycarbonyl)aminomethyl]-2-methoxy-2-methyl-1,3-dioxolane prepared by the same method as in EXAMPLE 3 was dissolved, and 0.2 ml of a 25% hydrogen bromide acetic acid solution was added to the resultant solution. After the addition, the mixture was stirred at room temperature for 2 hours. Then, the reaction solution was cooled with ice, and 5 ml of saturated aqueous sodium bicarbonate was added to the solution to terminate the reaction. After two times of extractions with 10 ml of ethyl acetate each, the whole organic layer was washed with 30 ml of saturated brine once and then dried over anhydrous magnesium sulfate. The organic layer was then concentrated under reduced pressure and dried in vacuum to produce 263 mg of a crude product. The crude product was purified by preparative thin-layer chromatography (developing solvent: ethyl acetate/n-hexane = 1/2) to produce 221 mg of (S)-2-(N-benzyloxycarbonyl)amino-1-(bromomethyl)ethyl acetate as a yellow oily compound. The overall yield from (S)-1-(N-benzyloxycarbonyl)amino-2,3-propanediol was 94%.
HPLC retention time: 8.5 min.
¹H NMR (400 MHz, CDCl₃) δ 7.34-7.26 (5H, m), 5.14 (2H, s), 5.10-5.01 (1H, m), 4.95 (1H, bs), 3.55-3.43 (4H, m), 2.08 (3H, s)

### (EXAMPLE 29) Preparation of (S)-2-(N-tert-butoxycarbonyl)amino-1-(chloromethyl)ethyl acetate

### [Preparation of Compound (5)]

In 15 ml of methylene chloride, 1.42 g (5.75 mmol) of (2R/S, 4S)-4-[(N-tert-butoxycarbonyl)aminomethyl]-2-methoxy-2-methyl-1,3-dioxolane prepared in EXAMPLE 7 was dissolved, and 812 mg (7.48 mmol, 130 mol%) of trimethylsilyl chloride was added dropwise to the resultant solution over 5 minutes. After the addition, the mixture was stirred at room temperature for 2 hours. Then, 10 ml of saturated aqueous sodium bicarbonate was added to the reaction solution to terminate the reaction. After three times of extractions with 10 ml of ethyl acetate each, the whole organic layer was washed with 30 ml of saturated brine once and then dried over anhydrous magnesium sulfate. The organic layer was then concentrated under reduced pressure and dried in vacuum to produce 1.29 g of (S)-2-(N-tert-butoxycarbonyl)amino-1-(chloromethyl)ethyl acetate. The overall yield from (S)-1-(N-tert-butoxycarbonyl)amino-2,3-propanediol was 89%.
HPLC analytical condition; column: Nacalai C8 4.6 mm I.D. × 250 mm, mobile phase: 10 mM {NaH₂PO₄-Na₂HPO₄} aqueous solution (pH = 6.8)/acetonitrile = 1/1 (vol/vol), flow rate: 1 ml/min, detection: UV 210 nm, column temperature: 40°C,
Injection volume: 10 µl
HPLC retention time: 7.8 min.
¹H NMR (400MHz, CDCl₃) δ 5.07 (1H, m), 4.73 (1H, m), 3.71-3.59 (2H, m), 3.47-3.40 (2H, m), 2.11 (3H, s), 1.44 (9H, s)

### (EXAMPLE 30) Preparation of (S)-2-(N-benzyloxycarbonyl)amino-1-(chloromethyl)ethyl acetate

### [Preparation of Compound (5)]

In 10 ml of toluene, 500 mg (4.44 mmol) of (S)-1-(N-benzyloxycarbonyl)amino-2,3-propanediol was suspended, and 1.00 g (6.22 mmol, 280 mol%) of trimethyl orthoacetate and 5.3 mg (0.0883 mmol, 1.99 mol%) of acetic acid were added to the resultant suspension. After the addition, the mixture was stirred at 110°C for 2 hours. After being cooled to room temperature, the mixture was concentrated from 10.9 g to 3.95 g in total weight under reduced pressure. Then, the residue was cooled with ice, and 343 mg (2.87 mmol, 130 mol%) of thionyl chloride was added dropwise to the residue over 3 minutes. After the addition, the resultant mixture was warmed to room temperature and then stirred for 2 hours. Then, 5 ml of saturated aqueous sodium bicarbonate was added to the reaction solution to terminate the reaction. After two times of extractions with 10 ml of toluene each, the whole organic layer was washed with 10 ml of saturated brine once. As a result of analysis of the organic layer by high-performance liquid chromatography, it was confirmed that (S)-2-(N-benzyloxycarbonyl)amino-1-(chloromethyl)ethyl acetate was produced from (S)-1-(N-benzyloxycarbonyl)amino-2,3-propanediol in an overall yield of 95%.

### (EXAMPLE 31) Preparation of (R)-2-(N-benzyloxycarbonyl)amino-1-(chloromethyl)ethyl acetate

### [Preparation of Compound (5)]

A toluene solution (352.35 g) of (2R/S, 4R)-4-[(N-benzyloxycarbonyl)aminomethyl]-2-methoxy-2-methyl-1,3-dioxolane prepared in EXAMPLE 11 was cooled with ice, and 32.48 g (0.259 mmol, 130 mol%) of thionyl chloride was added dropwise to the solution. After the addition, the mixture was warmed to room temperature and stirred for 2 hours. Then, 300 ml of water was added to the reaction solution to terminate the reaction, and the reaction solution was washed with 300 ml of water four times and 100 ml of a 5% sodium hydroxide aqueous solution once. After further washing with 200 ml of water, the organic layer was analyzed by high-performance liquid chromatography. As a result, it was confirmed that (R)-2-(N-benzyloxycarbonyl)amino-1-(chloromethyl)ethyl acetate was produced from (R)-1-(N-benzyloxycarbonyl)amino-2,3-propanediol in an overall yield of 99%. A part of the organic layer was concentrated and then measured by ¹H NMR to confirm the structure.
HPLC retention time: 8.7 min.
¹H NMR (400 MHz, CDCl₃) δ 7.36-7.31 (5H, m), 5.14 (2H, s), 5.14-5.01 (1H, m), 4.96 (1H, bs), 3.71-3.46 (4H, m), 2.08 (3H, s)

### (EXAMPLE 32) Preparation of (S)-1-(N-benzyloxycarbonyl)amino-3-chloro-2-propanol [Preparation of Compound (5)]

In 40 ml of dimethylformamide, 2.12 g of (2R/S, 4S)-4-[(N-benzyloxycarbonyl)aminomethyl]-1,3,2λ⁴-dioxathiolane-2-one prepared in EXAMPLE 12 was dissolved, and 1.56 g (36.7 mmol, 400 mol%) of lithium chloride was added to the resultant solution under ice-cooling, followed by stirring at 80°C for 12 hours. After cooling to room temperature, 20 ml of 1N hydrochloric acid was added to the reaction solution to terminate the reaction. After three times of extractions with 40 ml of ethyl acetate each, the whole organic layer was washed with 100 ml of water three times and 100 ml of saturated brine once, and then dried over anhydrous magnesium sulfate. The organic layer was then concentrated under reduced pressure and dried in vacuum to produce 1.84 g of a crude product. The product was purified by silica gel column chromatography (eluent: ethyl acetate/toluene = 1/3) to produce 1.72 g of (S)-1-(N-benzyloxycarbonyl)amino-3-chloro-2-propanol as a colorless oily compound in a yield of 90.5%.
HPLC retention time: 5.45 min.
¹H NMR (400 MHz, CDCl₃) δ 7.36-7.32 (5H, m), 5.12 (1H, bs), 5.11 (2H, s), 4.10-3.94 (1H, m), 3.60-3.50 (2H, m), 3.48-3.46 (1H, m), 3.34-3.28 (1H, m), 3.14 (1H, bs)

### (EXAMPLE 33) Preparation of (S)-1-(N-benzyloxycarbonyl)amino-3-bromo-2-propanol [Preparation of Compound (5)]

In 10 ml of acetonitrile, 263 mg of (2R/S, 4S)-4-[(N-benzyloxycarbonyl)aminomethyl]-1,3,2λ⁴-dioxathiolane-2-one prepared in EXAMPLE 14 was dissolved, and 200 mg (2.3 mmol, 237 mol%) of lithium bromide was added to the resultant solution at room temperature, followed by stirring under reflux for 7 hours. After cooling to room temperature, 74 mg of acetic acid was added to the reaction solution to terminate the reaction. After the solvent was distilled off under reduced pressure, 0.5 ml of 4N hydrochloric acid was added to the residue, and then extraction with a mixed solvent containing 6 ml of diethyl ether and 3 ml of hexane was performed twice. The whole organic layer was dried over anhydrous sodium sulfate and filtered through a silica gel short column, and the silica gel was washed with 3 ml of diethyl ether. The filtrate was then concentrated under reduced pressure and dried in vacuum to produce 250.3 mg of (S)-1-(N-benzyloxycarbonyl)amino-3-bromo-2-propanol as a colorless oily compound in a yield of 89.6%.
¹H NMR (400 MHz, CDCl₃) δ 7.38-7.33 (5H, m), 5.19 (1H, bs), 5.11 (2H, s), 3.95-3.94 (1H, m), 3.49-3.38 (3H, m), 3.36-3.29 (1H, m), 3.11 (1H, bs)

### (EXAMPLE 34) Preparation of (S)-1-(N-benzyloxycarbonyl)amino-3-bromo-2-propanol [Preparation of Compound (5)]

In 12 ml of 1,2-dimethoxyethane, 369.5 mg of (2R/S, 4S)-4-[(N-benzyloxycarbonyl)aminomethyl]-1,3,2λ⁴-dioxathiolane-2-one prepared in EXAMPLE 13 was dissolved, and 326 mg (3.75 mmol, 275 mol%) of lithium bromide was added to the resultant solution at room temperature, followed by stirring under reflux for 14 hours. After cooling to room temperature, the solvent was distilled off under reduced pressure, and 0.5 ml of 4N hydrochloric acid and 1 ml of water were added to the reaction mixture. Then, extraction with a mixed solvent containing 6 ml of diethyl ether and 3 ml of hexane was performed twice. The whole organic layer was dried over anhydrous sodium sulfate and filtered through a silica gel short column, and the silica gel was washed with 6 ml of diethyl ether. The filtrate was then concentrated under reduced pressure and dried in vacuum to produce 371.6 mg of (S)-1-(N-benzyloxycarbonyl)amino-3-bromo-2-propanol as a yellowish-orange oily compound in a yield of 94.7%.

### (EXAMPLE 35) Preparation of (R)-1-(N-benzyloxycarbonyl)amino-3-chloro-2-propanol [Preparation of Compound (5)]

In 20 ml of acetonitrile, 1271.6 mg of (2R/S, 4R)-4-[(N-benzyloxycarbonyl)aminomethyl]-1,3,2λ⁴-dioxathiolane-2-one prepared in EXAMPLE 15 was dissolved, and 620 mg (14.6 mmol, 312 mol%) of lithium chloride was added to the resultant solution at room temperature, followed by overnight stirring under reflux. During the course of reaction, the degree of reaction progress was confirmed by thin-layer chromatography after the elapse of 10 hours. As a result, a large amount of the raw material remained. Therefore, 20 ml of dioxane was added, and stirring under reflux was continued. After the elapse of 13 hours from the addition of dioxane, the degree of reaction progress was again measured. As a result, the raw material did not completely disappear, and thus 10 ml of dimethylformamide was added 27 hours after the start of reflux. Then, stirring under reflux was further continued for a total time of 34 hours. After the reaction mixture was cooled to room temperature, the solvent was distilled off under reduced pressure, and 50 ml of water containing 2 ml of 4N hydrochloric acid was added to the residue. Then, extraction with 25 ml of diethyl ether was preformed, and the aqueous layer was subjected to three times of extractions with 25 ml of diethyl ether each. The whole organic layer was dried over anhydrous sodium sulfate and filtered through a silica gel short column, and the silica gel was washed with 20 ml of diethyl ether. The filtrate was then concentrated under reduced pressure and dried in vacuum to produce 1018.5 mg of (R)-1-(N-benzyloxycarbonyl)amino-3-chloro-2-propanol as an orange oily compound in a yield of 89.2%.
¹H NMR (400 MHz, CDCl₃,) δ 7.36-7.32 (5H, m), 5.12 (1H, bs), 5.11 (2H, s), 4.10-3.94 (1H, m), 3.60-3.50 (2H, m), 3.48-3.46 (1H, m), 3.34-3.28 (1H, m), 3.14 (1H, bs)

### (EXAMPLE 36) Preparation of (S)-1-(N-benzoylamino)-3-bromo-2-propanol [Preparation of Compound (5)]

In 15 ml of tetrahydrofuran, 417.1 mg of (2R/S, 4S)-4-(N-benzoylaminomethyl)-1,3,2λ⁴-dioxathiolane-2-one prepared in EXAMPLE 16 was dissolved, and 615 mg (7.08 mmol, 410 mol%) of lithium bromide was added to the resultant solution at room temperature, followed by stirring under reflux for 5 hours. After the reaction mixture was cooled to room temperature, the solvent was distilled off under reduced pressure. Then, 5 ml of water containing 1 ml of 4N hydrochloric acid was added to the resulting concentrate, and extraction with 10 ml of diethyl ether was performed four times. The whole organic layer was dried over anhydrous sodium sulfate, concentrated under reduced pressure and dried in vacuum to produce 404.6 mg of (S)-1-(N-benzoylamino)-3-bromo-2-propanol as a lightly brownish-yellow solid compound in a yield of 90.7%.
¹H NMR (400 MHz, CDCl₃) δ 7.84-7.75 (2H, m), 7.57-7.41 (3H, m), 6.83, 6.67 (1H, bs×2), 4.11-4.02 (1H, m), 3.86-3.78 (1H, m), 3.62-3.54 (1H, m), 3.49-3.41 (2H, m)

### (EXAMPLE 37) Preparation of (R)-1-phthalimido-3-bromo-2-propanol [Preparation of Compound (5)]

In 70 ml of acetone, 3.41 g of (2R/S, 4R)-4-phthalimidomethyl-1,3,2λ⁴-dioxathiolane-2-one prepared in EXAMPLE 17 was dissolved, and 3.6 g (41.45 mmol, 357 mol%) of lithium bromide was added to the resultant solution at room temperature, followed by stirring under reflux for 9 hours. After the reaction mixture was cooled to room temperature, the solvent was distilled off under reduced pressure. Then, 40 ml of water containing 2 ml of concentrated hydrochloric acid was added to the resulting concentrate, and extraction with 50 ml of diethyl ether was performed. The aqueous layer was further subjected to two times of extractions with 25 ml of diethyl ether each. The whole organic layer was dried over anhydrous sodium sulfate, concentrated under reduced pressure and dried in vacuum to produce 3.46 g of (R)-1-phthalimido-3-bromo-2-propanol as a lightly yellowish white solid compound in a quantitative yield.
¹H NMR (400 MHz, CDCl₃) δ 7.89-7.83 (2H, m), 7.79-7.74 (2H, m), 4.20-4.13 (1H, m), 3.99-3.84 (2H, m), 3.58-3.45 (2H, m), 3.13 (1H, bs)

### (EXAMPLE 38) Preparation of (S)-2-[(N-benzyloxycarbonyl)aminomethyl]oxirane [Preparation of Compound (6)]

In 25 ml of methanol, 2.43 g (8.45 mmol) of (S)-2-(N-benzyloxycarbonyl)amino-1-(chloromethyl)ethyl acetate prepared by the same method as in EXAMPLE 19 was dissolved, and 2.50 g (17.00 mmol, 200 mol%) of potassium carbonate was added to the resultant solution, followed by stirring at room temperature for 2 hours. Then, 20 ml of a saturated aqueous ammonium chloride solution was added to the reaction solution, and extraction with 20 ml of ethyl acetate was performed. The organic layer was washed with 20 ml of saturated brine, dried over anhydrous magnesium sulfate, concentrated under reduced pressure and dried in vacuum to produce 1.75 g of a crude product. The crude product was purified by preparative thin-layer chromatography (developing solvent; ethyl acetate/toluene = 1/1) to produce 1.27 g of (S)-2-[(N-benzyloxycarbonyl)aminomethyl]oxirane as a colorless oily compound in a yield of 72%. The optical purity of the compound was 99.8% ee.
HPLC retention time: 5.0 min.
¹H NMR (400 MHz, CDCl₃) δ 7.38-7.30 (5H, m), 5.11 (2H, s), 5.00 (1H, bs), 3.65-3.59 (1H, m), 3.30-3.24 (1H, m), 3.15-3.05 (1H, m), 2.79-2.77 (1H, m), 2.60-2.59 (1H, m)

### (EXAMPLE 39) Preparation of (S)-2-[(N-benzyloxycarbonyl)aminomethyl]oxirane [Preparation of Compound (6)]

In 2 ml of methanol, 168 mg (0.535 mmol) of (S)-2-(N-benzyloxycarbonyl)amino-1-(chloromethyl)ethyl butylate prepared in EXAMPLE 20 was dissolved, and 90.2 mg (0.652 mmol, 120 mol%) of potassium carbonate was added to the resultant solution, followed by stirring at room temperature for 3 hours. Then, 5 ml of a saturated aqueous ammonium chloride solution was added to the reaction solution, and extraction with 10 ml of ethyl acetate was performed twice. The whole organic layer was washed with 20 ml of saturated brine, dried over anhydrous magnesium sulfate, concentrated under reduced pressure and dried in vacuum to produce 106 mg of a crude product. The analysis of the crude product by high-performance liquid chromatography showed that the product contained 93.2 mg of (S)-2-[(N-benzyloxycarbonyl)aminomethyl]oxirane in a yield of 84%. The optical purity of the compound was 99.7% ee.

### (EXAMPLE 40) Preparation of (S)-2-[(N-benzyloxycarbonyl)aminomethyl]oxirane [Preparation of Compound (6)]

In 2 ml of methanol, 133 mg (0.399 mmol) of (S)-2-(N-benzyloxycarbonyl)amino-1-(chloromethyl)ethyl benzoate prepared in EXAMPLE 21 was dissolved, and 93.6 mg (0.677 mmol, 170 mol%) of potassium carbonate was added to the resultant solution, followed by stirring at room temperature for 3 hours. Then, 5 ml of a saturated aqueous ammonium chloride solution was added to the reaction solution, and extraction with 10 ml of ethyl acetate was performed three times. The whole organic layer was washed with 20 ml of saturated brine, dried over anhydrous magnesium sulfate, concentrated under reduced pressure and dried in vacuum to produce 154 mg of a crude product. The analysis of the crude product by high-performance liquid chromatography showed that the product contained 111 mg of (S)-2-[(N-benzyloxycarbonyl)aminomethyl]oxirane in a yield of 85%. The optical purity of the compound was 99.3% ee.

### (EXAMPLE 41) Preparation of (S)-2-[(N-benzyloxycarbonyl)aminomethyl]oxirane [Preparation of Compound (6)]

In 1.7 ml of methanol, 163 mg (0.490 mmol) of (S)-2-(N-benzyloxycarbonyl)amino-1-(bromomethyl)ethyl acetate prepared in EXAMPLE 28 was dissolved, and 72.0 mg (0.545 mmol, 110 mol%) of potassium carbonate was added to the resultant solution, followed by stirring at room temperature for 1 hour. Then, 2 ml of a saturated aqueous ammonium chloride solution was added to the reaction solution, and extraction with 5 ml of ethyl acetate was performed twice. The whole organic layer was washed with 10 ml of saturated brine, dried over anhydrous magnesium sulfate, concentrated under reduced pressure and dried in vacuum to produce 85.0 mg of a crude product. The analysis of the crude product by high-performance liquid chromatography showed that the product contained 74.6 mg of (S)-2-[(N-benzyloxycarbonyl)aminomethyl]oxirane in a yield of 73%. The optical purity of the compound was 99.8% ee.

### (EXAMPLE 42) Preparation of (S)-2-[(N-tert-butoxycarbonyl)aminomethyl]oxirane [Preparation of Compound (6)]

In 6 ml of methanol, 567 mg (2.25 mmol) of (S)-2-(N-tert-butoxycarbonyl)amino-1-(chloromethyl)ethyl acetate prepared in EXAMPLE 29 was dissolved, and 347 mg (2.48 mmol, 110 mol%) of potassium carbonate was added to the resultant solution, followed by stirring at room temperature for 3 hours. Then, 10 ml of a saturated aqueous ammonium chloride solution was added to the reaction solution, and extraction with 10 ml of ethyl acetate was performed three times. The whole organic layer was washed with 20 ml of saturated brine, dried over anhydrous magnesium sulfate, concentrated under reduced pressure and dried in vacuum to produce 331 mg of (S)-2-[(N-tert-butoxycarbonyl)aminomethyl]oxirane as white crystals in a yield of 85%. The optical purity of the compound was 99.3% ee.
HPLC analytical condition; column: Nacalai C8 4.6 mm I.D. × 250 mm, mobile phase: 10 mM {NaH₂PO₄-Na₂HPO₄} aqueous solution (pH = 6.8)/acetonitrile = 1/1 (vol/vol), flow rate: 1 ml/min, detection: UV 210 nm, column temperature: 40°C, Injection volume: 10 µl
Optical purity analysis; column: CHIRALCEL OD-H 4.6 mm I.D. × 250 mm, mobile phase: n-hexane/isopropanol = 95/5
(vol/vol), flow rate: 0.5 ml/min, detection: UV 210 nm, column temperature: 40°C, Injection volume: 10 µl
HPLC retention time: 5.0 min.
¹H NMR (400 MHz, CDCl₃) δ 4.77 (1H, bs), 3.57-3.53 (1H, m), 3.28-3.3.18 (1H, m), 3.10-3.09 (1H, m), 2.80-2.78 (1H, m), 2.61-2.59 (1H, m), 1.45 (9H, s)

### (EXAMPLE 43) Preparation of (R)-2-[(N-benzyloxycarbonyl)aminomethyl]oxirane [Preparation of Compound (6)]

In 11 ml of methanol, 12.43 g (38.91 mmol) of a toluene solution of (R)-2-(N-benzyloxycarbonyl)amino-1-(chloromethyl)ethyl acetate prepared by the same method as in EXAMPLE 31 was dissolved, and 42 ml (42 mmol, 108 mol%) of a 1 mol/l sodium methoxide methanol solution was added dropwise to the resultant solution under ice cooling. After the addition, the resultant mixture was stirred for 4.5 hours. Then, 0.3 ml of acetic acid was added to the reaction solution to terminate the reaction, and the solution was concentrated under reduced pressure. Then, 30 ml of water and 80 ml of ethyl acetate were added for extraction, and the organic layer was washed with 30 ml of water. As a result of the analysis of the organic layer by high-performance liquid chromatography, it was confirmed that (R)-2-[(N-benzyloxycarbonyl)aminomethyl]oxirane was produced in a yield of 83%. The organic layer was then concentrated and purified by column chromatography (developing solvent; ethyl acetate/toluene = 1/2) to produce 3.01 g of (R)-2-[(N-benzyloxycarbonyl)aminomethyl]oxirane as a colorless oily substance. The colorless oily substance purified by column chromatography was crystallized from a mixed solvent containing ethyl acetate and n-hexane at 1:10, and then recrystallized from a mixed solvent containing ethyl acetate and n-hexane at 1:5 to produce 2.83 g of (R)-2-[(N-benzyloxycarbonyl)aminomethyl]oxirane as a white solid. The optical purity of the compound was 100% ee.
HPLC retention time: 5.0 min.
Melting point: 30 to 31°C
¹H NMR (400 MHz, CDCl₃) δ 7.38-7.30 (5H, m), 5.11 (2H, s), 5.00 (1H, bs), 3.65-3.59 (1H, m), 3.30-3.24 (1H, m), 3.15-3.05 (1H, m), 2.79-2.77 (1H, m), 2.60-2.59 (1H, m)

### (EXAMPLE 44) Preparation of (R)-2-[(N-benzyloxycarbonyl)aminomethyl]oxirane [Preparation of Compound (6)]

In 15 ml of methanol, 5.91 g (17.6 mmol) of a toluene solution of (R)-2-(N-benzyloxycarbonyl)amino-1-(chloromethyl)ethyl acetate prepared by the same method as in EXAMPLE 31 was dissolved, and 2.45 g (17.6 mmol, 100 mol%) of potassium carbonate was added to the resultant solution. After the addition, the resultant mixture was stirred under ice cooling for 5 hours. Then, 1 ml of acetic acid was added to the reaction solution to terminate the reaction, and the solution was concentrated. Then, 15 ml of water and 40 ml of ethyl acetate were added for extraction, and the organic layer was washed with 15 ml of water. As a result of the analysis of the organic layer by high-performance liquid chromatography, it was confirmed that (R)-2-[(N-benzyloxycarbonyl)aminomethyl]oxirane was produced in a yield of 90%.

### (EXAMPLE 45) Preparation of (S)-2-[(N-benzyloxycarbonyl)aminomethyl]oxirane [Preparation of Compound (6)]

In 13 ml of methanol, 1.32 g of (S)-1-(N-benzyloxycarbonyl)amino-3-chloro-2-propanol prepared in EXAMPLE 32 was dissolved, and 756 mg (5.47 mmol, 100 mol%) of potassium carbonate was added to the resultant solution under ice cooling. After the addition, the resultant mixture was stirred at the same temperature for 100 minutes and at room temperature for 4 hours. During the course of reaction, 76.2 mg (0.55 mmol, 10 mol%) and 75.3 mg (0.54 mmol, 10 mol%) of potassium carbonate were added after stirring at room temperature for 1 hour and 2 hours, respectively. Then, 408.2 mg (6.8 mmol) of acetic acid was added to the reaction solution, and the insoluble solid was filtered off and washed with 10 ml of ethyl acetate. The filtrate was concentrated under reduced pressure to produce a concentrate which was then purified by silica gel column chromatography (eluent: toluene → diethyl ether/hexane =1/10 → diethyl ether/hexane = 1/2 → diethyl ether/hexane =1/1) to produce 1011.1 mg of (S)-2-[(N-benzyloxycarbonyl)aminomethyl]oxirane as a colorless oily compound in a yield of 90.1%. The optical purity of the compound was 100% ee.
¹H NMR (400 MHz, CDCl₃) δ 7.38-7.30 (5H, m), 5.11 (2H, s), 5.00 (1H, bs), 3.65-3.59 (1H, m), 3.30-3.24 (1H, m), 3.15-3.05 (1H, m), 2.79-2.77 (1H, m), 2.60-2.59 (1H, m)

### (EXAMPLE 46) Preparation of (S)-2-[(N-benzyloxycarbonyl)aminomethyl]oxirane [Preparation of Compound (6)]

In 6 ml of methanol, 250.3 mg and 371.6 mg of (S)-1-(N-benzyloxycarbonyl)amino-3-bromo-2-propanol prepared in EXAMPLE 33 and EXAMPLE 34, respectively, were dissolved, and 343.6 mg (2.49 mmol, 115 mol%) of potassium carbonate was added to the resultant solution at room temperature. After the addition, the resultant mixture was stirred at the same temperature for 30 minutes. Then, 166.7 mg (2.77 mmol) of acetic acid was added to the reaction solution, and the solvent was distilled off under reduced pressure. Then, a mixed solvent containing 5 ml of diethyl ether and 5 ml of hexane was added to the resultant concentrate. The insoluble solid was filtered off by passing through a silica gel short column, and the solid and silica gel were washed with a mixed solvent containing 5 ml of diethyl ether and 5 ml of hexane three times. The filtrate was concentrated under reduced pressure and dried in vacuum to produce 389.8 mg of (S)-2-[(N-benzyloxycarbonyl)aminomethyl]oxirane as a yellow oily compound in a yield of 87.2%. The optical purity of the compound was 100% ee.

### (EXAMPLE 47) Preparation of (R)-2-[(N-benzyloxycarbonyl)aminomethyl]oxirane [Preparation of Compound (6)]

In 13 ml of toluene, 1018.5 mg of (R)-1-(N-benzyloxycarbonyl)amino-3-chloro-2-propanol prepared in EXAMPLE 35 was dissolved, and 190.8 mg (4.77 mmol, 114 mol%) of 60% oily sodium hydride was added to the resultant solution under ice cooling. Immediately after the addition, the reaction system was put into a nitrogen atmosphere, and the mixture was stirred at the same temperature for 90 minutes. Then, 34 mg (0.57 mmol) of acetic acid was added to the reaction solution to terminate the reaction. The reaction mixture was purified by silica gel column chromatography (eluent: hexane → diethyl ether/hexane =1/5 → diethyl ether/hexane = 1/3 → diethyl ether/hexane =1/2 → diethyl ether/hexane = 1/1) to produce 594.8 mg of (R)-2-[(N-benzyloxycarbonyl)aminomethyl]oxirane as a yellow oily compound in a yield of 68.7%. The optical purity of the compound was 99.9% ee.
¹H NMR (400 MHz, CDCl₃) δ 7.38-7.30 (5H, m), 5.11 (2H, s), 5.00 (1H, bs), 3.65-3.59 (1H, m), 3.30-3.24 (1H, m), 3.15-3.05 (1H, m), 2.79-2.77 (1H, m), 2.60-2.59 (1H, m)

### (EXAMPLE 48) Preparation (S)-2-(N-benzoylaminomethyl)oxirane [Preparation of Compound (6)]

In 10 ml of methanol, 404.6 mg of (S)-1-(N-benzoylamino)-3-bromo-2-propanol prepared in EXAMPLE 36 was dissolved, and 225.1 mg (1.63 mmol, 104 mol%) of potassium carbonate was added to the resultant solution under ice cooling, followed by stirring at the same temperature for 2.5 hours. After reaction for 2 hours, 50.8 mg (0.37 mmol, 23 mol%) of potassium carbonate was added for accelerating the reaction. After further stirring at room temperature for 1 hour, 2 droplets of acetic acid were added to the reaction solution, and the solvent was distilled off under reduced pressure. Then, a mixed solvent containing 5 ml of ethyl acetate and 1.7 ml of hexane was added to the resultant concentrate. The insoluble solid was rapidly filtered off through a silica gel short column, and the solid and the silica gel were washed with a mixed solvent containing 15 ml of ethyl acetate and 5 ml of hexane two times. The filtrate was concentrated under reduced pressure and dried in vacuum to produce 232.2 mg of (S)-2-(N-benzoylaminomethyl)oxirane as an orangish yellow solid compound in a yield of 83.6%. The optical purity of the compound was 100% ee.
¹H NMR (400 MHz, CDCl₃) δ 7.84-7.75 (2H, m), 7.56-7.40 (3H, m), 6.49 (1H, bs), 3.98-3.89 (1H, m), 3.58-3.48 (1H, m), 3.25-3.20 (1H, m), 2.85-2.81 (1H, m), 2.68-2.64 (1H, m)

### (EXAMPLE 49) Preparation of (R)-2-(phthalimidomethyl)oxirane

### [Preparation of Compound (6)]

In 10 ml of tetrahydrofuran, 269 mg (6.73 mmol, 196 mol%) of 60% oily sodium hydride was suspended, and 1023 mg of (R)-1-phthalimido-3-bromo-2-propanol prepared in EXAMPLE 37 was added to the resultant suspension at a time. Immediately after the addition, the reaction system was put into a nitrogen atmosphere, and the mixture was stirred at the same temperature for 18 hours. Then, a solution prepared by dissolving 249.1 mg (4.15 mmol) of acetic acid in 10 ml of ethyl acetate was added to the reaction solution to terminate the reaction. Furthermore, 10 ml of ethyl acetate and 20 ml of water were added to the reaction solution, and the product was extracted and separated. The aqueous layer was subjected to extraction with 20 ml of ethyl acetate twice. The whole organic layer was dried over anhydrous sodium sulfate, concentrated under reduced pressure, and dried in vacuum to produce a lightly yellowish white solid. The solid was purified by silica gel column chromatography (eluent: methylene chloride/hexane =1/2 → diethyl ether/hexane = 1/2 → diethyl ether → diethyl ether/methylene chloride = 2/1) to produce 632.2 mg of (R)-2-(phthalimidomethyl)oxirane as a white solid compound in a yield of 90.6%. The optical purity of the compound was 98.9% ee.

The optical purity was analyzed under the following conditions:
Column: CHIRALPAK AD-H 4.6 mm I.D. × 250 mm
Mobile phase: n-hexane/isopropanol = 90/10 (vol/vol)
Flow rate: 1.0 ml/min
Detection: UV 254 nm
Column temperature: 30°C
Injection volume: 3 µl
¹H NMR (400 MHz, CDCl₃) δ 7.90-7.85 (2H, m), 7.78-7.72 (2H, m), 4.00-3.93 (1H, m), 3.85-3.78 (1H, m), 3.27-3.23 (1H, m), 2.83-2.79 (1H, t), 2.71-2.67 (1H, m)

### (EXAMPLE 50) Preparation (R)-2-(phthalimidomethyl)oxirane

### [Preparation of Compound (6)]

In 25 ml of toluene, 2.43 g of (R)-1-phthalimido-3-bromo-2-propanol prepared in EXAMPLE 37 was suspended, and 2.16 g (11.2 mmol, 137 mol%) of a 28% sodium methoxide methanol solution was added to the resultant suspension at room temperature under a nitrogen atmosphere. After stirring at the same temperature for 1.5 hours, 25 ml of water was added to the reaction solution to terminate the reaction. The reaction mixture was subjected to a separating operation, and the aqueous layer was further subjected to extraction with 25 ml of ethyl acetate once. The whole organic layer was dried over anhydrous sodium sulfate, concentrated under reduced pressure and dried in vacuum to produce 0.94 g of (R)-2-(phthalimidomethyl)oxirane as a lightly yellowish white solid compound in a yield of 56.7%. The optical purity of the compound was 98.9% ee. Industrial Applicability

According to the present invention having the above-described constitution, optically active 1-substituted amino-2,3-epoxypropanes useful as intermediates for preparing agricultural chemicals and medical products can be effectively and advantageously prepared by an industrial process. Also, novel synthetic intermediates for preparing the optical active epoxypropanes can be provided.

## Claims

1. A process for preparing an optically active 1-substituted amino-2,3-epoxypropane represented by formula (6): 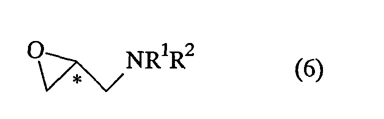 (wherein * represents an asymmetric carbon atom, R¹ and R² independently represent a hydrogen atom or a carbamate-, acyl- or aroyl-type amino protecting group, or R¹ and R² represent together an imide-type amino protecting group), the process comprising reacting an optically active 1-substituted amino-2,3-propanediol represented by formula (1): 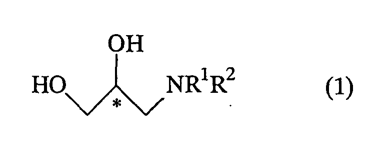 (wherein * represents an asymmetric carbon atom, and R¹ and R² represent the same as the above) with a compound represented by formula (2) or (3):
R³C(OR⁴)₃ (2)
(wherein R³ represents a hydrogen atom, an alkyl group having 1 to 6 carbon atoms, an aryl group having 6 to 10 carbon atoms, or a substituted or unsubstituted aralkyl group having 7 to 10 carbon atoms, and R⁴ represents an alkyl group having 1 to 6 carbon atoms),
SOY₂ (3)
(wherein Y represents a halogen atom or a lower alkoxy group) to produce an optically active compound represented by formula (4): 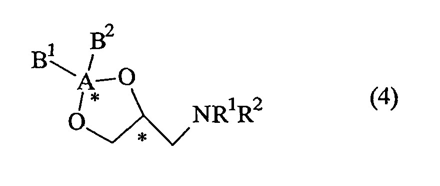 [wherein * represents an asymmetric carbon atom or an asymmetric sulfur atom, A represents a carbon atom or a sulfur atom, B¹ represents R³ (representing the same as the above), and B² represents OR⁴ (wherein R⁴ represents the same as the above) or B¹ and B² represent together an oxygen atom, and R¹ and R² represent the same as the above]; opening the ring of the compound represented by formula (4) to produce an optically active compound represented by formula (5): 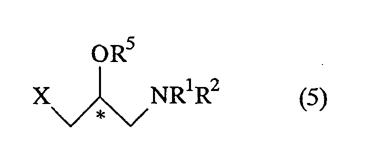 [wherein * represents an asymmetric carbon atom, X represents a halogen atom, R⁵ represents COR³ (wherein R³ represents the same as the above) or a hydrogen atom, and R¹ and R² represent the same as the above]; and further subjecting the compound represented by formula (5) to ring closure in the presence of a base.

2. A process for preparing an optically active compound represented by formula (4): 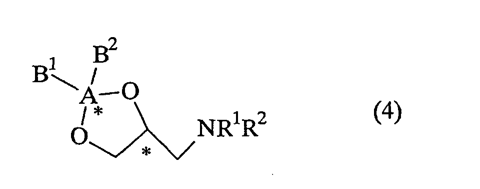 [wherein * represents an asymmetric carbon atom or an asymmetric sulfur atom, A represents a carbon atom or a sulfur atom, B¹ represents R³ (representing the same as the above), and B² represents OR⁴ (wherein R⁴ represents the same as the above) or B¹ and B² represent together an oxygen atom, and R¹ and R² represent the same as the above], the process comprising reacting an optically active 1-substituted amino-2,3-propanediaol represented by formula (1): 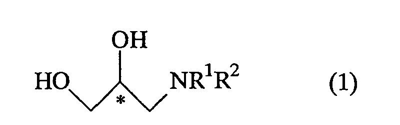 (wherein * represents an asymmetric carbon atom, R¹ and R² independently represent a hydrogen atom or a carbamate-, acyl- or aroyl-type amino protecting group, or R¹ and R² represent together an imide-type amino protecting group) with a compound represented by formula (2) or (3):
R³C(OR⁴)₃ (2)
(wherein R³ represents a hydrogen atom, an alkyl group having 1 to 6 carbon atoms, an aryl group having 6 to 10 carbon atoms, or a substituted or unsubstituted aralkyl group having 7 to 10 carbon atoms, and R⁴ represents an alkyl group having 1 to 6 carbon atoms),
SOY₂ (3)
(wherein Y represents a halogen atom or a lower alkoxy group).

3. An optically active compound represented by formula (4): 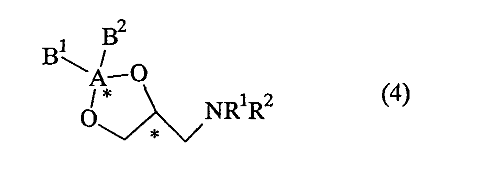 [wherein * represents an asymmetric carbon atom or an asymmetric sulfur atom, A represents a carbon atom or a sulfur atom, B¹ represents R³ (representing the same as the above), and B² represents OR⁴ (wherein R⁴ represents the same as the above) or B¹ and B² represent together an oxygen atom, and R¹ and R² represent the same as the above].

4. A process for preparing an optically active compound represented by formula (5): 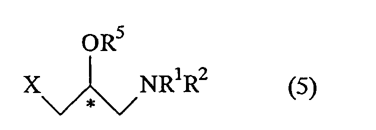 [wherein * represents an asymmetric carbon atom, X represents a halogen atom, R⁵ represents COR³ (wherein R³ represents the same as the above) or a hydrogen atom, and R¹ and R² represent the same as the above], the process comprising opening the ring of an optically active compound represented by formula (4): 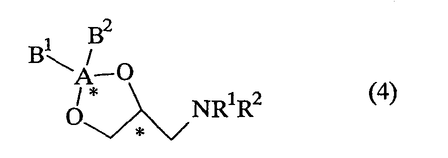 [wherein * represents an asymmetric carbon atom or an asymmetric sulfur atom, A represents a carbon atom or a sulfur atom, B¹ represents R³ (representing the same as the above), and B² represents OR⁴ (wherein R⁴ represents the same as the above) or B¹ and B² represent together an oxygen atom, and R¹ and R² represent the same as the above].

5. A process for preparing an optically active 1-substituted amino-2,3-epoxypropane represented by formula (6): 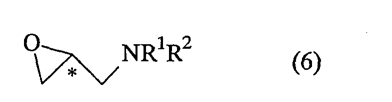 (wherein * represents an asymmetric carbon atom, and R¹ and R² represent the same as the above], the process comprising preparing an optically active compound represented by formula (5): 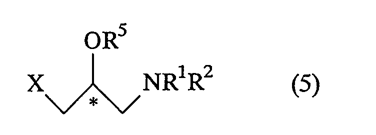 [wherein * represents an asymmetric carbon atom, X represents a halogen atom, R⁵ represents COR³ (wherein R³ represents the same as the above) or a hydrogen atom, and R¹ and R² represent the same as the above], and then subjecting the compound to ring closure in the presence of a base.
